# EUROPEAN PATENT APPLICATION

(11) **EP 4 431 042 A1**
(43) Date of publication of application: **18.09.2024**
(21) Application number: 24162195.2
(22) Date of filing: 07.03.2024
(51) Int. Cl.: A61B 34/20, A61B 90/30

(54) **MEDICAL SUPPORT DEVICE, AND OPERATION METHOD AND OPERATION PROGRAM OF MEDICAL SUPPORT DEVICE**

(30) Priority: 15.03.2023 JP 2023041051
(71) Applicant: FUJI-FILM Corporation, Minato-ku Tokyo 106-8620 (JP)
(72) Inventor: MASUMOTO, Jun, Tokyo, 106-8620 (JP); SASUGA, Saeko, Tokyo, 106-8620 (JP)
(74) Representative: Hughes, Andrea Michelle

(57) **Abstract**

A processor (41) of a medical support device (11) is configured to acquire a captured image (23) that is captured by an extracorporeal camera (15) provided outside a body of a subject and in which a medical device (13) whose insertion portion (13A) is inserted into the body of the subject and a marker (M1) which is provided at a portion of the medical device excluding the insertion portion and is image-recognizable are included in an imaging range; derive position and posture information including at least one of a position or a posture of the medical device in the captured image (23) based on the marker (M1); and execute a control of displaying, on a display unit (16), a composite image in which medical support information is superimposed at a position specified in the captured image based on the position and posture information.

## Description

### BACKGROUND OF THE INVENTION

### 1. Field of the Invention

A technique of the present disclosure relates to a medical support device, and an operation method and an operation program of a medical support device.

### 2. Description of the Related Art

In the endoscopic surgery, an ultrasound image of an inside of a liver is acquired using an ultrasound probe, and a puncture target which is a position of a tumor is ascertained in the ultrasound image. However, even in a case where the puncture target is displayed in the ultrasound image, since the ultrasound image is an image showing an inside of an organ in the body, it is difficult to know from which position and in which direction the puncture needle may be inserted in the body surface.

In order to solve such a problem, a technique for supporting the puncture is used. For example, US8688196B discloses a technique that displays a position and a posture of a distal end of a puncture needle inserted into a body, in real time, by using a magnetic navigation system using the puncture needle in which a magnetic position sensor is provided at the distal end. EP3136940A discloses a technique that uses a laser pointer that irradiates a distal end of an ultrasound probe provided with a guide groove for guiding an insertion of a puncture needle, with laser light, toward a body surface side along an inclination of the guide groove.

### SUMMARY OF THE INVENTION

US8688196B has a problem in that a large-scale device such as a magnetic navigation system is required. In EP3136940A, since the laser light is only emitted in a direction corresponding to an inclination of a guide groove, there is a problem in that the degree of freedom of display with respect to a display content and a display position of support information is low. As described above, in the related art, in a case where a medical device having an insertion portion into a body is used, there is a problem in that it is not possible to display medical support information at an appropriate position corresponding to a position or a posture of the medical device in a simple configuration and a higher degree of freedom of display.

A technique according to the present invention provides a medical support device, an operation method and an operation program capable of displaying medical support information at an appropriate position corresponding to a position or a posture of a medical device in a simple configuration and a higher degree of freedom of display.

According to a first aspect of the present invention, there is provided a medical support device comprising a processor, in which the processor is configured to: acquire a captured image that is captured by an extracorporeal camera provided outside a body of a subject and in which a medical device whose insertion portion is inserted into the body of the subject and a marker which is provided at a portion of the medical device excluding the insertion portion and is image-recognizable are included in an imaging range; derive position and posture information including at least one of a position or a posture of the medical device in the captured image based on the marker; and execute a control of displaying, on a display unit, a composite image in which medical support information is superimposed at a position specified in the captured image based on the position and posture information.

It is preferable that the medical device is used for an endoscopic surgery, has an insertion portion to be inserted into the body from a hole formed in a body surface of the subject on a distal end side, and has the marker provided on a proximal end side that is not inserted into the body, and the processor is configured to derive the position and posture information of the insertion portion of the medical device based on the marker.

It is preferable that the processor is configured to derive the position and posture information of the insertion portion based on the marker and dimensional information of the medical device.

It is preferable that, in a case where the medical device is configured to change a position and a posture of the insertion portion by operating an operation portion provided outside the body, the processor is configured to acquire an operation amount of the operation portion in addition to the dimensional information, and the acquired operation amount is used for deriving the position and posture information of the insertion portion.

It is preferable that the medical device is a first medical device having an intracorporeal camera for imaging the inside of the body, and in a case where the insertion portion of the first medical device is set as a first insertion portion, the marker of the first medical device is set as a first marker, the captured image is set as a first captured image, and the position and posture information is set as first position and posture information, and further, a second medical device, which is different from the first medical device and is provided with a second insertion portion to be inserted into the body and a second marker that is image-recognizable in the second insertion portion, is used for surgery together with the first medical device, the processor is configured to: acquire a second captured image which is captured by the intracorporeal camera and in which the second insertion portion and the second marker are included in an imaging range; derive second position and posture information including at least one of a position or a posture of the second insertion portion in the second captured image based on the second marker; and execute a control of displaying, on the display unit, a composite image in which the medical support information is superimposed on the first captured image at a position specified in the first captured image based on the first position and posture information and the second position and posture information.

It is preferable that the medical device is a medical probe that is configured to observe an internal structure of an organ.

It is preferable that the first medical device is an endoscope, and the second medical device is a medical probe that is configured to observe an internal structure of an organ.

It is preferable that the medical support information is insertion support information of a treatment tool that is inserted from outside the body toward a target position in the organ observed through the medical probe, and is the insertion support information including at least one of an insertion position or an insertion route.

It is preferable that the medical probe is an ultrasound probe.

It is preferable that the treatment tool is a puncture needle.

It is preferable that the insertion position is displayed by a mark indicating a position on the body surface of the subject at which the puncture needle is inserted.

It is preferable that the insertion route is indicated by a line.

It is preferable that the medical probe is provided in the insertion portion and has a guide groove for guiding insertion of the treatment tool into the target position by engaging with the treatment tool, and the processor is configured to specify a position at which the insertion support information is superimposed on the captured image based on a relative positional relationship between the second marker and the guide groove.

It is preferable that the processor is configured to specify a position at which the insertion support information is superimposed on the captured image based on the target position specified in an internal image of the organ acquired by the medical probe, and a correlation between a coordinate system of the internal image and a coordinate system of the captured image which are derived based on the position and posture information.

It is preferable that in a case where the insertion support information is set in a three-dimensional image of the organ acquired in advance before surgery by simulation before the surgery, the processor is configured to superimpose the insertion support information on the first captured image using a correlation between a coordinate system of an internal image of the organ acquired by the medical probe and a coordinate system of the three-dimensional image, and a correlation between a coordinate system of the internal image and a coordinate system in the second captured image which are derived based on the second position and posture information.

It is preferable that the extracorporeal camera is provided on a proximal end side of the treatment tool and is configured to output the captured image as a video image, and the processor is configured to display a line indicating the insertion route in the video image as the insertion support information.

It is preferable that the treatment tool is a puncture needle, and an imaging optical axis of the extracorporeal camera is disposed along an axial direction of the puncture needle.

It is preferable that the captured images are at least two captured images captured from different viewpoints, and the processor is configured to derive the insertion position on the body surface of the subject by obtaining an intersection of the insertion routes shown in the two captured images.

According to another aspect of the present invention, there is provided an operation method of a medical support device including a processor, the operation method comprising: via the processor, acquiring a captured image that is captured by an extracorporeal camera provided outside a body of a subject and in which a medical device whose insertion portion is inserted into the body of the subject and a marker which is provided at a portion of the medical device excluding the insertion portion and is image-recognizable are included in an imaging range; derive position and posture information including at least one of a position or a posture of the medical device in the captured image based on the marker; and execute a control of displaying, on a display unit, a composite image in which medical support information is superimposed at a position specified in the captured image based on the position and posture information.

According to another aspect of the present invention, there is provided an operation program of a medical support device that causes a computer to function as a medical support device, the operation program causing the computer to: acquire a captured image that is captured by an extracorporeal camera provided outside a body of a subject and in which a medical device whose insertion portion is inserted into the body of the subject and a marker which is provided at a portion of the medical device excluding the insertion portion and is image-recognizable are included in an imaging range; derive position and posture information including at least one of a position or a posture of the medical device in the captured image based on the marker; and execute a control of displaying, on a display unit, a composite image in which medical support information is superimposed at a position specified in the captured image based on the position and posture information.

### BRIEF DESCRIPTION OF THE DRAWINGS

Fig. 1 is a diagram showing an outline of a medical support system including a medical support device.
Fig. 2 is a diagram showing a state of an inside of a body in endoscopic surgery.
Fig. 3 is a diagram showing an ultrasound probe and an ultrasound image.
Fig. 4 is a diagram showing a hardware configuration of the medical support device.
Fig. 5 is a flowchart showing a procedure of entire medical support processing.
Fig. 6 is a diagram showing a method of deriving position and posture information based on a first marker located outside a body.
Fig. 7 is a diagram showing a relationship between a position and a posture of the first marker and a medical device.
Fig. 8 is another diagram showing a relationship between the position and the posture of the first marker and the medical device.
Fig. 9 is a diagram showing a method of deriving position and posture information based on a second marker located inside a body.
Fig. 10 is a diagram showing coordinate conversion between an intracorporeal surgical field image and an extracorporeal surgical field image.
Fig. 11 is a diagram showing a change in display due to movement of a viewpoint of an extracorporeal camera.
Fig. 12 is a diagram showing stereo imaging.
Fig. 13 is a diagram showing a method using a correlation between the ultrasound image and the extracorporeal surgical field image.
Fig. 14 is a diagram showing a second embodiment.
Fig. 15 is a diagram showing an example of a laparotomy.
Fig. 16 is a diagram showing an example in which the extracorporeal camera is provided in the robot.
Fig. 17 is a diagram showing a method of creating a presurgical 3D image.
Fig. 18 is a diagram showing a method of creating presurgical preparation information.
Fig. 19 is a diagram showing a method of creating an ultrasound 3D image.
Fig. 20 is a diagram showing a method using a correlation between the ultrasound 3D image and the presurgical 3D image.

### DESCRIPTION OF THE PREFERRED EMBODIMENTS

### First embodiment

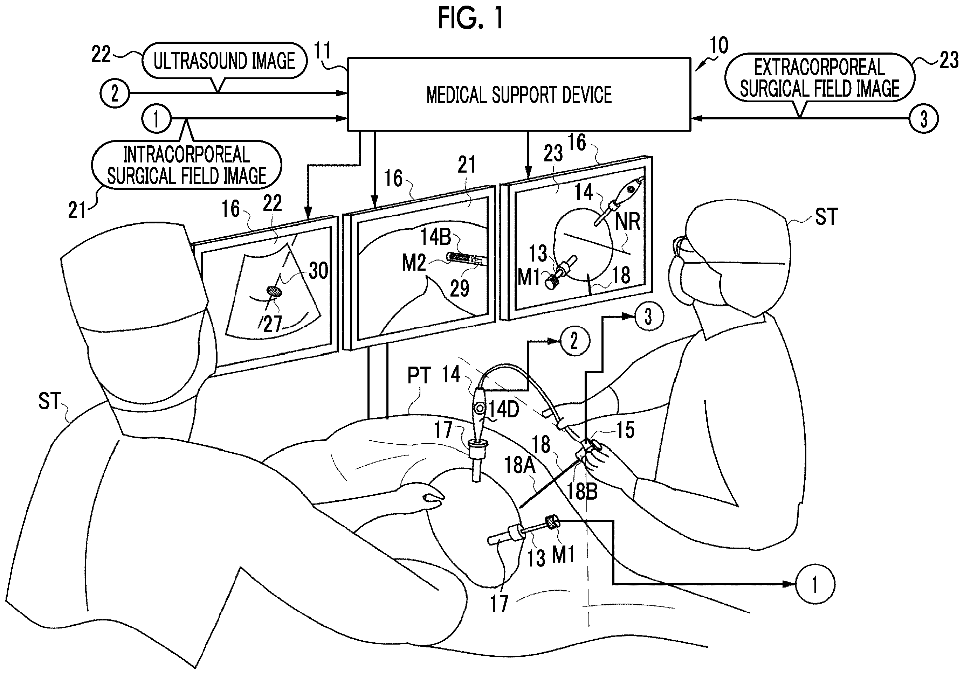

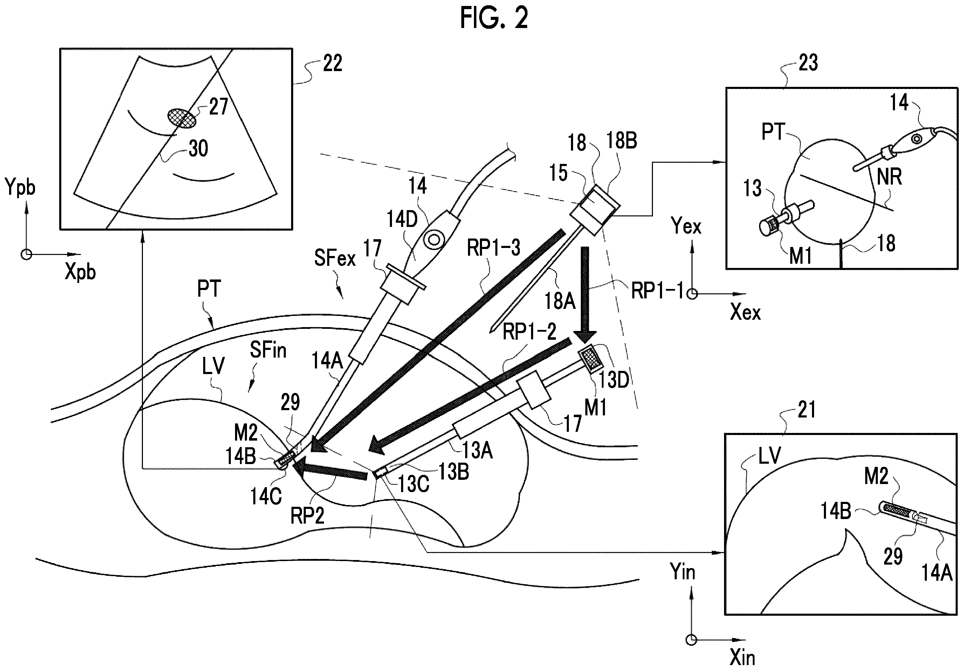

As shown in Figs. 1 and 2, a medical support system 10 is used, for example, in a case where an endoscopic surgery is performed on a patient PT using an endoscope 13 or the like. The endoscopic surgery is surgery that is performed by making a small hole in a body of the patient PT and inserting a medical device such as the endoscope 13 from the hole, unlike a laparotomy. The medical support system 10 provides a visual field of a surgical field to a medical staff ST including a doctor. Here, the surgical field is described by distinguishing an intracorporeal surgical field SFin in the body of the patient PT and an extracorporeal surgical field SFex which is a region outside the body corresponding to the intracorporeal surgical field SFin. As will be described later, both an inside-the-body and an outside-the-body visual fields are provided to the medical staff ST. Further, the medical support system 10 provides support information for supporting medical care, such as surgery and an examination, in addition to the visual field of the surgical field. Since the medical support system 10 has a function of providing support information in real time during the surgery, the medical support system 10 is also called a surgical navigation system or the like. As an example, the medical support system 10 comprises a medical support device 11, an endoscope 13, an ultrasound probe 14, an extracorporeal camera 15, and a display 16. The medical support device 11 is an example of a "medical support device" according to the technique of the present disclosure, and the patient PT is an example of a "subject".

The medical support device 11 is communicably connected to the endoscope 13, the ultrasound probe 14, the extracorporeal camera 15, and the display 16. In the endoscopic surgery, a part of the endoscope 13 and the ultrasound probe 14 including distal end portions is inserted into the body through a trocar 17. The trocar 17 is an insertion tool provided with an insertion hole for inserting the endoscope 13 or the like and a valve provided in the insertion hole to prevent gas leakage. In the endoscopic surgery, the trocar 17 is used for insertion into the body, such as the endoscope 13 and the ultrasound probe 14, because pneumoper itoneum is performed by injecting carbon dioxide gas into the abdominal cavity.

In the example, a target site of the surgery is a liver LV, and Fig. 2 shows a state in which the endoscope 13 and the ultrasound probe 14 are inserted into the abdomen of the patient PT. Reference numeral 18 is a puncture needle. The puncture needle 18 is, for example, a treatment tool that punctures a lesion, such as a tumor 27 included in an organ, as a puncture target and cauterizes the lesion. As will be described below, in the present example, as an example of surgery, a treatment of necrotizing a tumor 27 by visualizing the tumor 27 of the liver LU with the ultrasound image 22 and cauterizing the visualized tumor 27 with the puncture needle 18 is will be described. The puncture needle 18 has a needle portion 18A and a grip portion 18B provided on a proximal end side of the needle portion 18A. The grip portion 18B is provided with an extracorporeal camera 15. An imaging optical axis of the extracorporeal camera 15 is disposed along an axial direction of the puncture needle 18. As a result, a direction of the viewpoint of the extracorporeal camera 15 and a direction of the needle tip of the puncture needle 18 substantially match each other.

The endoscope 13 has an insertion portion 13A to be inserted into the body of a patient PT, and a camera 13C and a light source for illumination (such as a light emitting diode (LED)) are built in a distal end portion 13B of the insertion portion 13A. The endoscope 13 is, for example, a rigid endoscope in which the insertion portion 13A is rigid, and also called a thoracoscope or the like because it is often used for observing an abdominal cavity. The camera 13C includes an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image. The endoscope 13 is an example of a "medical device" and a "first medical device" according to the technique of the present disclosure, and the insertion portion 13A is an example of a "first insertion portion". The camera 13C is an example of an "intracorporeal camera" according to the technique of the present disclosure.

The endoscope 13 optically images the intracorporeal surgical field SFin including a target site (liver LV in the present example) in the body of the patient PT by the camera 13C. The endoscope 13 is connected to an image processing processor (not shown) for an endoscope, and the image processing processor performs signal processing on an imaging signal output from the image sensor to generate an intracorporeal surgical field image 21 of the intracorporeal surgical field SFin. The intracorporeal surgical field image 21 is an example of a "second captured image captured by the intracorporeal camera" according to the technique of the present disclosure.

As the illumination light of the endoscope 13, special light such as ultraviolet rays and infrared light may be used, but visible light such as white light is used. As the illumination light of the endoscope 13, the special light such as the ultraviolet rays and the infrared light may be used. As the special light, light limited to a specific wavelength, such as short-wavelength narrow-band light obtained by narrowing down light in a short wavelength range such as an ultraviolet range may be used. The intracorporeal surgical field image 21 captured by the endoscope 13 is transmitted to the medical support device 11 in real time through an image processing processor for an endoscope. The intracorporeal surgical field image 21 is displayed on the display 16 as a video image. In Fig. 2, reference numerals Xin and Yin indicate a coordinate system of the intracorporeal surgical field image 21.

Further, a grip portion 13D is provided on a proximal end side of the endoscope 13. The grip portion 13D is provided with a marker M1 capable of image-recognizing. The marker M1 is a marker that is recognizable in an optically captured image, that is, an optically detectable marker. The marker M1 is provided in a portion excluding the insertion portion 13A to be inserted into the body. The grip portion 13D is an example of a "portion excluding the insertion portion" according to the technique of the present disclosure. That is, the marker M1 is provided at a position visible from the outside the body, and is imaged by the extracorporeal camera 15 in the present example. The medical support device 11 uses the marker M1 to estimate the position and the posture of the insertion portion 13A to be inserted into the body.

For example, the marker M1 is formed of a pattern having a morphological feature such as a lattice pattern or a dot pattern. The estimation of the position and the posture using the marker M1 will be described below. The marker M1 is an example of a "marker" and a "first marker" according to the technique of the present disclosure.

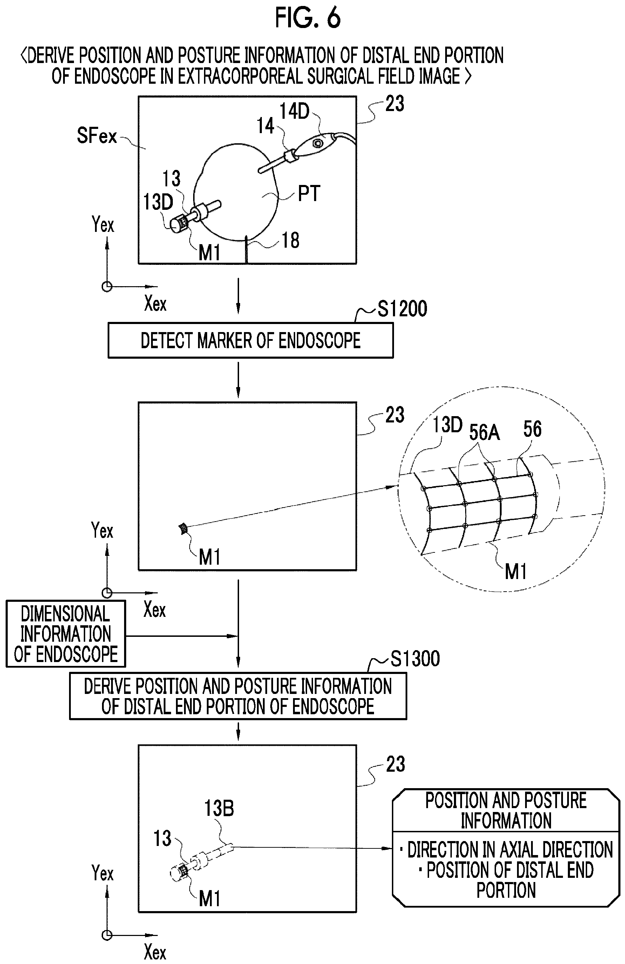

The ultrasound probe 14 has an insertion portion 14A to be inserted into the body of the patient PT, and an operation portion 14D on a proximal end side of the insertion portion 14A, similarly to the endoscope 13. An ultrasound transducer 14C is built in a distal end portion 14B of the insertion portion 14A. The ultrasound transducer 14C transmits an ultrasonic wave to a target site and receives a reflected wave reflected by the target site. The ultrasound probe 14 is connected to an image processing processor (not shown) for an ultrasound probe. The image processing processor for an ultrasound probe performs image reconstruction processing based on the reflected wave on the basis of a signal corresponding to the relfected wave received by the ultrasound probe 14. An ultrasound image 22 (see Fig. 6) indicating an internal structure of the target site scanned by the ultrasound probe 14 is generated by the image reconstruction processing. The ultrasound image 22 is a so-called brightness (B)-mode image in which the internal structure from a surface layer to deep layer to which the ultrasonic wave reaches the target site is visualized as brightness information. The ultrasound image 22 visualizes the internal structure of a target site that cannot be observed in the intracorporeal surgical field image 21 obtained by optical imaging.

The ultrasound probe 14 is, for example, a convex type that radially transmits ultrasonic waves, and acquires a fan-shaped image with the ultrasound transducer 14C as a base point. The ultrasound image 22 corresponding to an operation position is captured along a scanning direction by scanning the ultrasound probe 14. The ultrasound image 22 is transmitted to the medical support device 11 in real time through the image processing processor for an ultrasound probe. The ultrasound image 22 is displayed on the display 16 as a video image. In Fig. 2, reference numerals Xpb and Ypb indicate a coordinate system of the ultrasound image 22.

In the ultrasound probe 14, the distal end portion 14B of the insertion portion 14A is provided with a guide groove 29 that guides the insertion of the puncture needle 18.

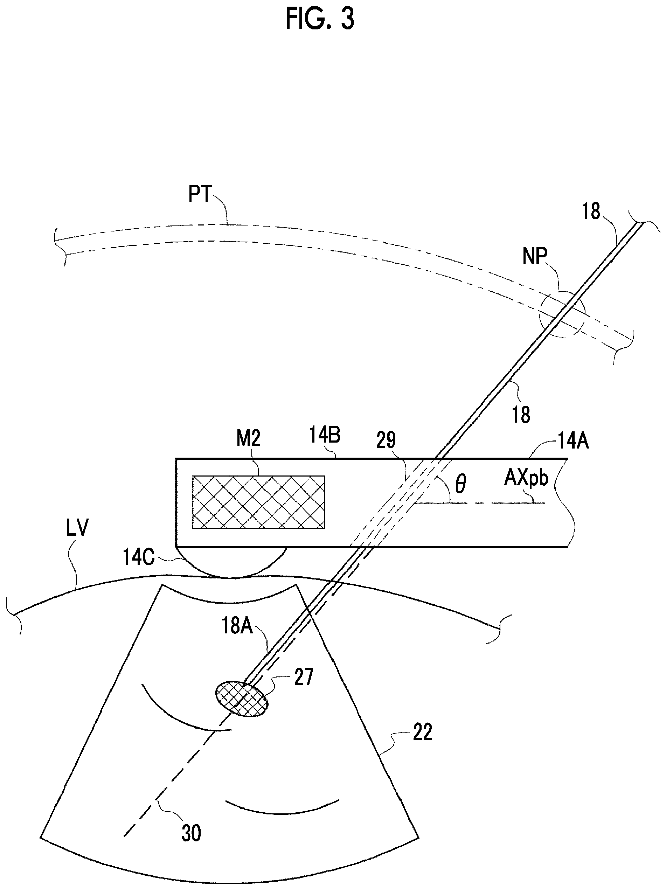

As shown in Fig. 3, the guide groove 29 is provided on the proximal end side with respect to the ultrasound transducer 14C at the distal end portion 14B. The guide groove 29 is inclined at an angle of θ with respect to an axial direction AXpb of the distal end portion 14B. The guide groove 29 is rearwardly inclined toward the proximal end side such that the needle tip of the puncture needle 18 inserted from a body surface side of the patient PT faces the distal end side where the ultrasound transducer 14C is disposed. The puncture needle 18 is inserted while the tumor 27 is confirmed by the ultrasound image 22. Since a region visualized by the ultrasound image 22 is a radial region with the ultrasound transducer 14C as a base point, the guide groove 29 is inclined, and thus the needle tip of the puncture needle 18 can be directed toward the tumor 27 visualized by the ultrasound image 22.

The distal end portion 14B is provided with a marker M2. The marker M2 is a marker capable of image-recognizing in the same manner as the marker M1. The marker M2 can be imaged by the camera 13C of the endoscope 13. The medical support device 11 uses the marker M2 to estimate the position and the posture of the distal end portion 14B of the insertion portion 14A. In the present example, the marker M2 is used as information for estimating the position and the posture of the guide groove 29 provided in the distal end portion 14B.

For example, the marker M2 is formed of a pattern having a morphological feature such as a lattice pattern or a dot pattern, as in the marker M1. The estimation of the position and the posture using the marker M2 will be described below. The marker M2 is an example of a "second marker" according to the technique of the present disclosure. The ultrasound probe 14 is an example of a "second medical device" according to the technique of the present disclosure, and the insertion portion 14A is an example of a "second insertion portion".

The extracorporeal camera 15 is an optical camera capable of optical imaging, similar to the camera 13C of the endoscope 13, and includes an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image. The extracorporeal camera 15 is an example of an "extracorporeal camera" according to the technique of the present disclosure.

The extracorporeal camera 15 optically images an extracorporeal surgical field SFex including the endoscope 13 and the ultrasound probe 14. The extracorporeal camera 15 is connected to an image processing processor (not shown), and the image processing processor performs signal processing on an imaging signal output from the image sensor to generate an extracorporeal surgical field image 23 of the extracorporeal surgical field SFex. The extracorporeal surgical field image 23 is transmitted to the medical support device 11 in real time through the image processing processor. The extracorporeal surgical field image 23 is displayed on the display 16 as a video image. In Fig. 2, reference numerals Xex and Yex indicate a coordinate system of the extracorporeal surgical field image 23. The extracorporeal surgical field image 23 is an example of a "captured image captured by the extracorporeal camera" and a "first captured image" according to the technique of the present disclosure.

The medical support device 11 acquires an intracorporeal surgical field image 21 from the endoscope 13, acquires an ultrasound image 22 from the ultrasound probe 14, and acquires an extracorporeal surgical field image 23 from the extracorporeal camera 15. As shown in Fig. 1, three displays 16 are prepared as an example, and three images of the intracorporeal surgical field image 21, the ultrasound image 22, and the extracorporeal surgical field image 23 are displayed on each display 16.

In a case where the ultrasound probe 14 is inserted into the intracorporeal surgical field SFin, the ultrasound probe 14 is reflected in the intracorporeal surgical field image 21. The medical support device 11 outputs the intracorporeal surgical field image 21, in which the insertion portion 14A (including the distal end portion 14B and the marker M2) of the ultrasound probe 14 is reflected, to the display 16. The visual field of the intracorporeal surgical field SFin of the patient PT is provided to the medical staff ST through the screen of the display 16.

As shown in Fig. 3, in the ultrasound image 22, it is possible to display a puncture line 30 in a superimposed manner along an inclination angle θ of the guide groove 29 as information indicating an insertion route of the puncture needle 18. The puncture line 30 is, an extension line of the guide groove 29, so to speak. The puncture line 30 is used as a guide in a case where the puncture needle 18 is inserted, and for example, the ultrasound probe 14 is positioned such that the puncture line 30 and the tumor 27 overlap with each other in the ultrasound image 22.

In a case where the operation portion 14D of the ultrasound probe 14, and the grip portion 13D and the marker M1 of the endoscope 13 enter the visual field of the extracorporeal camera 15, the extracorporeal surgical field image 23 including the operation portion 14D of the ultrasound probe 14, and the grip portion 13D and the marker M1 of the endoscope 13 in the imaging range are imaged. The medical support device 11 outputs such an extracorporeal surgical field image 23 to the display 16. The visual field of the extracorporeal surgical field SFex of the patient PT is provided to the medical staff ST through the screen of the display 16.

As described above, even in a case where the puncture line 30 is displayed in the ultrasound image 22, it is difficult to understand at which position and in what posture the puncture needle 18 is to be inserted from the outside the body because the ultrasound image 22 is an internal image of the organ. Specifically, as shown in Fig. 3, it is difficult to understand an insertion route including an insertion position NP and an insertion angle on the body surface of the patient PT. Therefore, the medical support device 11 displays at least one of an insertion route NR or the insertion position NP of the puncture needle 18 in a superimposed manner on the extracorporeal surgical field SFex. As a result, insertion support information, such as the insertion route NR and the insertion position NP, is provided to the medical staff ST. Such insertion support information is an example of "insertion support information" and "medical support information" according to the technique of the present disclosure.

Conceptually, the functions of the medical support device 11 are as follows. That is, as shown in Fig. 2, the medical support device 11 grasps a relative positional relationship RP1-1 between the extracorporeal camera 15 and the grip portion 13D from the extracorporeal surgical field image 23 which is captured by the extracorporeal camera 15 and includes the marker M1 of the endoscope 13 in the imaging range. Specifically, the position and the posture of the marker M1 in the extracorporeal surgical field image 23 captured by the extracorporeal camera 15 are grasped. Since dimensional information of the endoscope 13 is known, a relative positional relationship RP1-2 such as the distance between the marker M1 and the camera 13C provided in the distal end portion 13B of the endoscope 13 is known. The medical support device 11 grasps the position and the posture of the camera 13C from the position and the posture of the marker M1 based on the positional relationship RP1-2. The medical support device 11 grasps the position and posture of the camera 13C to grasp an imaging direction (more specifically, a direction of an imaging optical axis) of the camera 13C.

Then, the medical support device 11 grasps a relative positional relationship RP2 between the camera 13C and the marker M2 from the intracorporeal surgical field image 21 which is captured by the camera 13C and includes the marker M2 of the ultrasound probe 14 in the imaging range. Since dimensional information of the ultrasound probe 14 is known, a relative positional relationship between the marker M2 and the guide groove 29 is also known. By using these relative positional relationships RP1-1, RP1-2, RP2, and the like, it is possible to grasp a relative positional relationship RP1-3 between the extracorporeal camera 15 and the guide groove 29. The medical support device 11 derives position and posture information of the distal end portion 13B of the insertion portion 13A of the endoscope 13 in the extracorporeal surgical field image 23 based on the marker M1, and derives position and posture information of the guide groove 29 in the intracorporeal surgical field image 21 based on marker M2. Then, the medical support device 11 superimposes insertion support information including any of the insertion route NR and the insertion position NP of the puncture needle 18 on the position specified in the extracorporeal surgical field image 23, based on the position and posture information of the camera 13C of the distal end portion 13B of the endoscope 13 and the position and posture information of the guide groove 29. As a result, the insertion support information is provided to the medical staff ST through the display 16 on which the extracorporeal surgical field image 23 is displayed. Hereinafter, the details will be described.

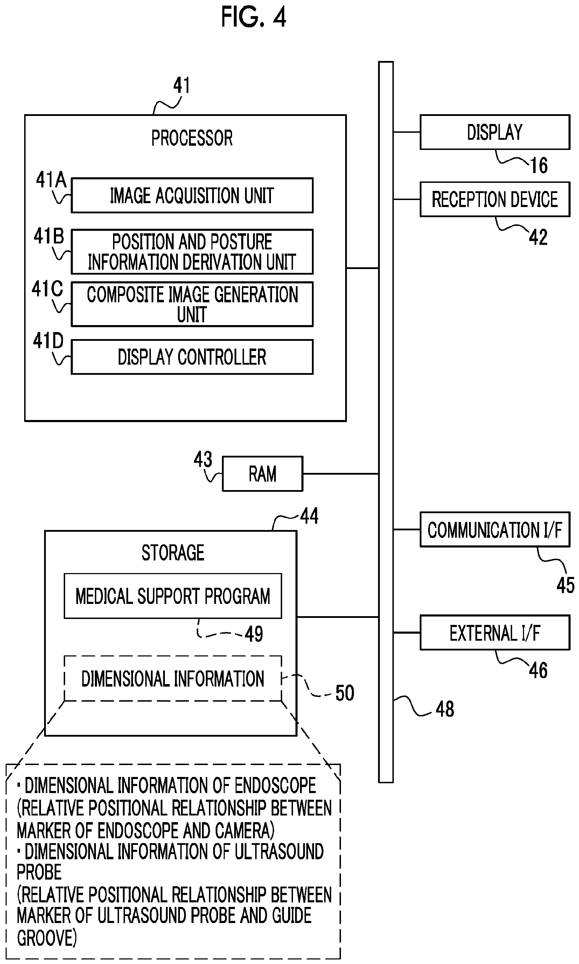

In Fig. 4, an example of the hardware configuration of the medical support device 11 is shown. The medical support device 11 is an example of a "medical support device" and a "computer" according to the technique of the present disclosure, and comprises a processor 41, a reception device 42, a display 16, a random access memory (RAM) 43, a storage 44, a communication I/F 45, and an external I/F 46. Each unit is connected to a bus 48 and can communicate with each other.

The medical support device 11 is operated by an operator, such as the medical staff ST, through the reception device 42. The reception device 42 has a keyboard, a mouse, and the like (not shown), and receives an instruction from the operator. The reception device 42 may be a device that receives a touch input, such as a touch panel, a device that receives a voice input, such as a microphone, a device that receives a gesture input, such as a camera, or the like.

Examples of the display 16 include an electro-luminescence (EL) display and a liquid crystal display. As described above, there are three displays 16, and various types of information are displayed on each display 16 in addition to the intracorporeal surgical field image 21, the ultrasound image 22, and the extracorporeal surgical field image 23.

The processor 41 is, for example, a central processing unit (CPU), and integrally controls each unit of the medical support device 11 following a control program and executes various kinds of processing following various kinds of application programs.

The storage 44 is a nonvolatile storage device that stores various programs, various parameters, and the like. Examples of the storage 44 include a hard disk drive (HDD) and a solid state drive (SSD). In the storage 44, a medical support program 49 that causes a computer to function as the medical support device 11 is stored.

The RAM 43 is a memory where information is temporarily stored, and is used as a work memory by the processor 41. An example of the RAM 43 includes a dynamic random access memory (DRAM) or a static random access memory (SRAM).

The communication I/F 45 is connected to a network (not shown), such as a local area network (LAN) and/or a wide area network (WAN), and performs transmission control following a communication protocol defined in various kinds of wired or wireless communication standards.

The external I/F 46 is, for example, a universal serial bus (USB) interface, and is used for connection to peripheral equipment, such as a printer and a memory card.

The processor 41 executes medical support processing by reading out the medical support program 49 from the storage 44 and executing the medical support program 49 on the RAM 43. The medical support processing is realized by the processor 41 operating as an image acquisition unit 41A, a position and posture information derivation unit 41B, a composite image generation unit 41C, and a display controller 41D. The medical support program 49 is an example of an "operation program of a medical support device" according to the technique of the present disclosure.

Dimensional information 50 includes dimensional information of the endoscope 13 and the ultrasound probe 14. Specifically, the dimensional information of the endoscope 13 includes information indicating a relative positional relationship between the marker M1 and camera 13C, such as a linear distance indicating an interval between the marker M1 and the camera 13C of the endoscope 13, a relationship of relative postures, and the like. Specifically, the dimensional information of the ultrasound probe 14 is information indicating a relative positional relationship between the marker M2 and the guide groove 29, such as a linear distance indicating an interval between the marker M2 and the guide groove 29 of the ultrasound probe 14, a relationship of relative postures, and the like. Further, the dimensional information of the ultrasound probe 14 also includes the inclination angle θ of the guide groove 29 with respect to the axial direction.

The image acquisition unit 41A executes image acquisition processing of acquiring the intracorporeal surgical field image 21, an ultrasound image group 22G, and the extracorporeal surgical field image 23. For example, the image acquisition unit 41A acquires the intracorporeal surgical field image 21 or/and the ultrasound image group 22G from a device including a processor of the endoscope 13 or/and a processor of the ultrasound probe 14 through the external I/F 46 or the communication I/F 45. The medical support device 11 may include the processor of the endoscope 13 or/and the processor of the ultrasound probe 14. Similarly, the image acquisition unit 41A acquires the extracorporeal surgical field image 23 from a device including the processor of the extracorporeal camera 15 through the external I/F 46 or the communication I/F 45. The medical support device 11 may include a processor of the extracorporeal camera 15.

The position and posture information derivation unit 41B executes processing of deriving position and posture information including the position and the posture of the camera 13C of the endoscope 13 based on the marker M1 and processing of deriving the position and posture information including the position and the posture of the guide groove 29 of the ultrasound probe 14 based on the marker M2.

The composite image generation unit 41C specifies a position at which the insertion support information is superimposed on the extracorporeal surgical field image 23 based on the derived position and posture information, and generates a composite image in which the insertion support information is superimposed on the specified position. The extracorporeal surgical field image 23 on which information such as the insertion route NR is superimposed is an example of a "composite image" according to the technique of the present disclosure.

The display controller 41D executes control of displaying the intracorporeal surgical field image 21, the ultrasound image 22, and the extracorporeal surgical field image 23 on the display 16. In the following description, a case where a composite image in which the insertion route NR is superimposed on the extracorporeal surgical field image 23 is generated and the generated composite image is displayed is also referred to as "display in a superimposed manner".

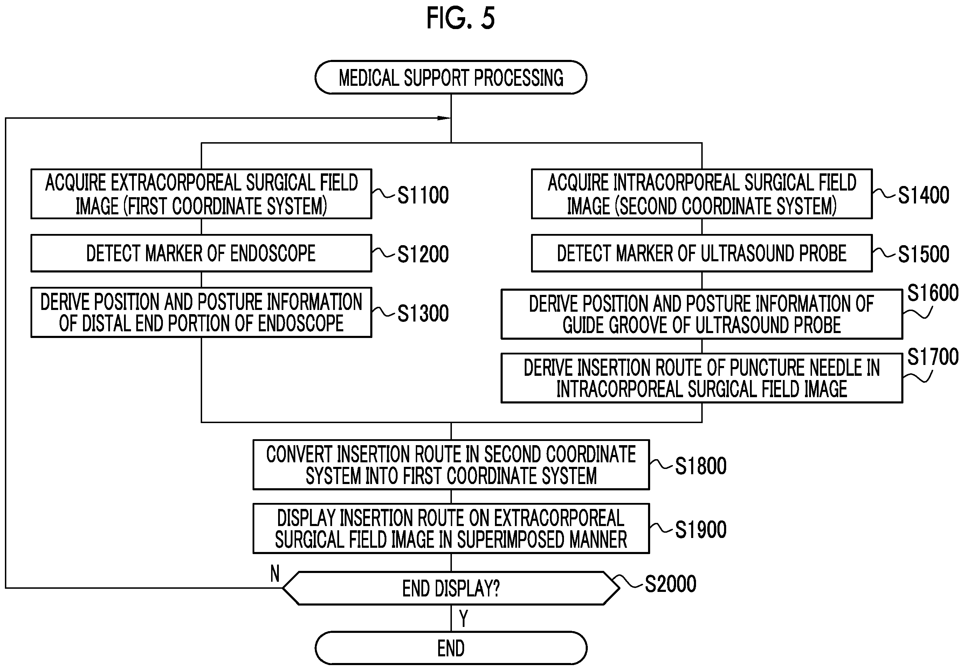

The processing executed by the processor 41 of the medical support device 11 of the present disclosure will be specifically described with reference to Figs. 5 to 12. Fig. 5 is a flowchart showing an overall processing procedure, and individual processing is described in Figs. 6 to 12.

As shown in Fig. 5, in the medical support processing, the processor 41 acquires the extracorporeal surgical field image 23 captured by the extracorporeal camera 15 in step S 1100. Then, in step S1200, the processor 41 detects the marker M1 of the endoscope 13 from the extracorporeal surgical field image 23. In step S1300, the processor 41 derives position and posture information of the distal end portion 13B of the endoscope 13 provided with the camera 13C based on the marker M1.

On the other hand, in step S 1400, the processor 41 acquires the intracorporeal surgical field image 21 captured by the camera 13C of the endoscope 13. Then, in step S1500, the processor 41 detects the marker M2 of the ultrasound probe 14 from the intracorporeal surgical field image 21. In step S1600, the processor 41 derives position and posture information of the guide groove 29 of the ultrasound probe 14 based on the marker M2. In step S1700, the processor 41 derives the insertion route NR of the puncture needle 18 in the intracorporeal surgical field image 21 based on the position and posture information of the guide groove 29.

Here, a coordinate system of the extracorporeal surgical field image 23 (hereinafter, referred to as a first coordinate system) and a coordinate system of the intracorporeal surgical field image 21 (hereinafter, referred to as a second coordinate system) are different from each other. In step S1800, the processor 41 converts the insertion route NR of the puncture needle 18 in the intracorporeal surgical field image 21 of the second coordinate system into the first coordinate system of the extracorporeal surgical field image 23. Then, in step S1900, the processor 41 displays the insertion route NR converted into the first coordinate system in a superimposed manner on the extracorporeal surgical field image 23. The processor 41 repeats such processing until the display is ended (step S2000).

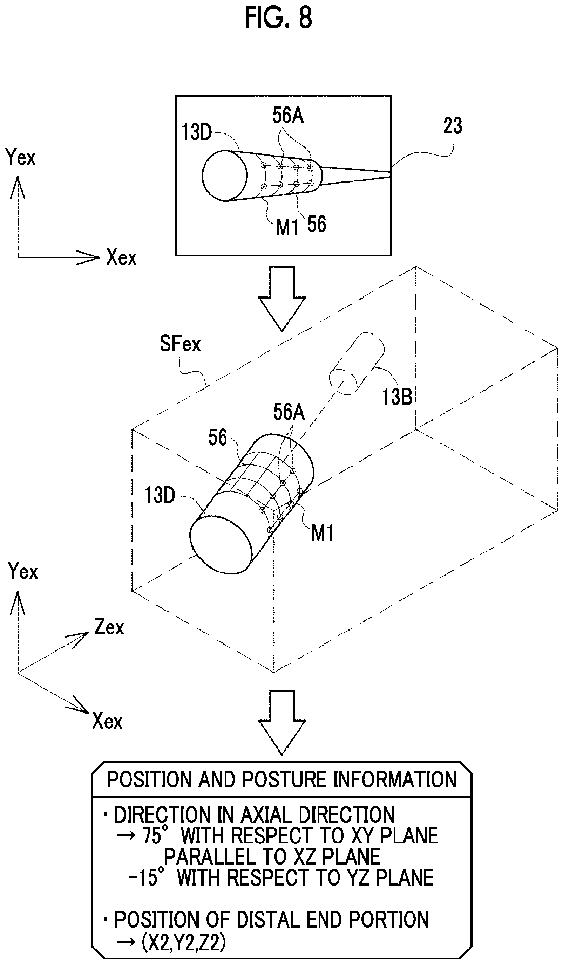

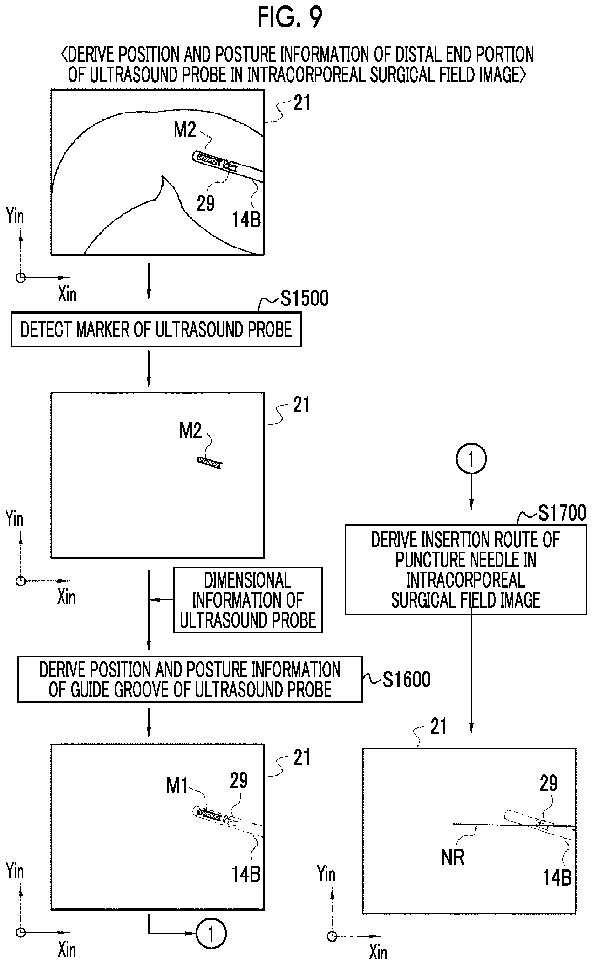

Hereinafter, each processing step will be described in more detail. First, Fig. 6 is a diagram conceptually showing the detection of the marker M1 in step S1200 shown in Fig. 5 and the derivation of position information of the distal end portion 13B of the endoscope 13 in step S1300. As a supplementary description of Fig. 6, the description will be made with reference to Fig. 8 and Fig. 9 as appropriate.

As shown in Fig. 6, in step S1200, the processor 41 detects the marker M1 based on the extracorporeal surgical field image 23 in the first coordinate system using, for example, an image processing method such as pattern matching. As an example, the marker M1 is a marker of a lattice pattern 56 that is configured with a first line extending in an axial direction of the grip portion 13D of the endoscope 13 and a second line formed in a circumferential direction orthogonal to the axial direction and along an outer peripheral surface of the grip portion 13D. A lattice interval of the lattice pattern 56 is constant. Reference numeral 56A indicates a plurality of intersections between the first line and the second line. The processor 41 detects the marker M1 by searching for a morphological feature of the marker M1, such as the lattice pattern 56, from the extracorporeal surgical field image 23. Of course, the marker M1 may be detected using an artificial intelligence technique instead of a rule-based method such as pattern matching.

The processor 41 specifies a region in which the marker M1 is present in the extracorporeal surgical field image 23 by detecting the marker M1. Then, in step S1300, first, the position and the posture of the grip portion 13D are estimated based on the morphological feature, such as the lattice pattern 56 of the marker M1. Next, the processor 41 estimates the position and the posture of the distal end portion 13B based on the estimated position and posture of the grip portion 13D and the dimensional information of the endoscope 13. A specific method thereof is as follows.

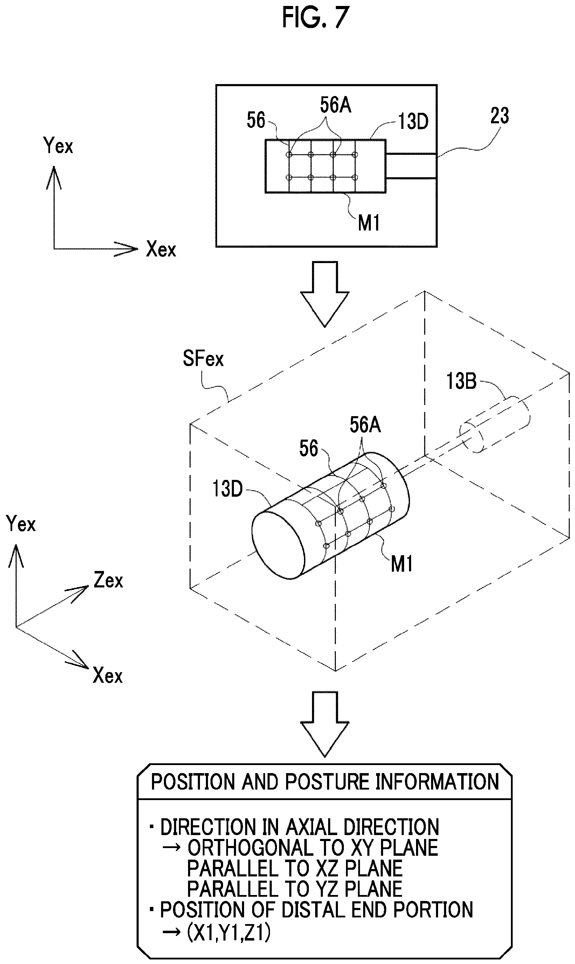

Figs. 7 and 8 show a correspondence relationship between the position and the posture of the grip portion 13D having the marker M1 in the extracorporeal surgical field image 23 and the position and the posture of the grip portion 13D in the extracorporeal surgical field SFex defined as a three-dimensional space. A Zex-axis of the extracorporeal surgical field SFex, which is a three-dimensional space, is a direction parallel to the imaging optical axis of the extracorporeal camera 15, and an Xex-Yex plane (hereinafter, simply referred to as an XY plane) is a plane parallel to the imaging surface of the extracorporeal camera 15 and is a surface, and the XY plane is orthogonal to the imaging optical axis. The XY plane is parallel to a screen of the extracorporeal surgical field image 23. Figs. 7 and 8 are conceptual diagrams, and the distal end portion 13B present in the body is also shown in the extracorporeal surgical field SFex.

Fig. 7 shows a state in which the axial direction of the grip portion 13D of the endoscope 13 is orthogonal to the imaging optical axis of the extracorporeal camera 15 in the extracorporeal surgical field SFex of the three-dimensional space (more specifically, a state in which the axial direction is parallel to the Xex axis). In a case where the grip portion D of the endoscope 13 is in such a posture, in the extracorporeal surgical field image 23, the lines orthogonal to the lattice pattern 56 of the marker M1 are in a parallel state with the Xex axis and the Yex axis, respectively, and intervals between the adjacent intersections 56A are the same.

On the other hand, Fig. 8 shows a state in which the axial direction of the grip portion 13D of the endoscope 13 in the extracorporeal surgical field SFex in the three-dimensional space is not orthogonal to the imaging optical axis but is inclined in a depth direction parallel to the imaging optical axis. The posture shown in Fig. 8 is a state in which the grip portion 13D is rotated by about -15° about the Y-axis from the posture shown in Fig. 7. In a case where the grip portion 13D is in such a posture, in the extracorporeal surgical field image 23, a line that extends in the peripheral direction among the lines of the lattice pattern 56 of the marker M1 is shortened as the line is farther from the extracorporeal camera 15 in the depth direction. Further, the intervals between the adjacent intersections 56A are also shortened as the intervals between the intersections 56A are further away from the extracorporeal camera 15.

In this way, a form of the marker M1 reflected in the extracorporeal surgical field image 23 is changed depending on the posture of the grip portion 13D. The processor 41 detects the posture of the grip portion 13D of the endoscope 13 based on the form of the marker M1 in the extracorporeal surgical field image 23. Further, a relative positional relationship between the marker M1 and the distal end portion 13B of the endoscope 13 is known. The processor 41 estimates the position and the posture of the distal end portion 13B of the endoscope 13 in the extracorporeal surgical field SFex based on the position and posture of the grip portion 13D and the dimensional information of the endoscope 13. The extracorporeal surgical field image 23 is a projection image in which the extracorporeal surgical field SFex is projected from one viewpoint. Therefore, by estimating the position and the posture of the distal end portion 13B in the extracorporeal surgical field SFex, the position and the posture of the distal end portion 13B in the extracorporeal surgical field image 23 can be estimated. In a case where the position and the posture of the distal end portion 13B can be grasped, the imaging direction of the camera 13C in the distal end portion 13B can also be estimated based on the dimensional information of the endoscope 13.

In the position and posture information of the example of Fig. 7, for example, the information is that the direction of the distal end portion 13B of the endoscope 13 in the axial direction is parallel to the XY plane and the XZ plane, and orthogonal to the YZ plane. The coordinates of the position a reference point of the distal end portion 13B are information such as X1, Y1, and Z1. In the position and posture information of the example of Fig. 8, for example, the information is that the direction of the distal end portion 13B in the axial direction is 75° with respect to the XY plane, parallel to the XZ plane, and -15° with respect to the YZ plane. The coordinates of the position the reference point of the distal end portion 13B are information such as X2, Y2, and Z2.

As shown in Fig. 9, in step S1500, the processor 41 detects the marker M2 based on the intracorporeal surgical field image 21 in the second coordinate system, for example, using the image processing method such as the pattern matching in the same manner as in the detection method of the marker M1. The pattern of the marker M2 is also, for example, the same lattice pattern 56 as the marker M1.

In a case where the region in which the marker M2 is present in the intracorporeal surgical field image 21 is specified by the detection of the marker M2, in step S1600, the processor 41 first estimates the position and the posture of the distal end portion 14B of the ultrasound probe 14 based on the morphological feature, such as the lattice pattern 56 of the marker M2. The processor 41 estimates the position and the posture of the guide groove 29 based on the estimated position and posture of the distal end portion 14B and the dimensional information of the ultrasound probe 14. The method of estimating the position and the posture of the guide groove 29 based on the marker M2 is the same as the methods shown in Figs. 7 and 8 with the marker M1 as an example.

Then, in step S1700, the processor 41 derives the insertion route NR of the puncture needle 18 in the intracorporeal surgical field image 21 based on the position and the posture of the guide groove 29. The processor 41 derives the insertion route NR which is an extension line of the guide groove 29 in the intracorporeal surgical field image 21 in accordance with the dimensional information such as the inclination angle θ of the guide groove 29. For example, a line segment having a position of guide groove 29 as a base point and an angle of the inclination angle θ of the guide groove 29 from the base point is set as the insertion route NR.

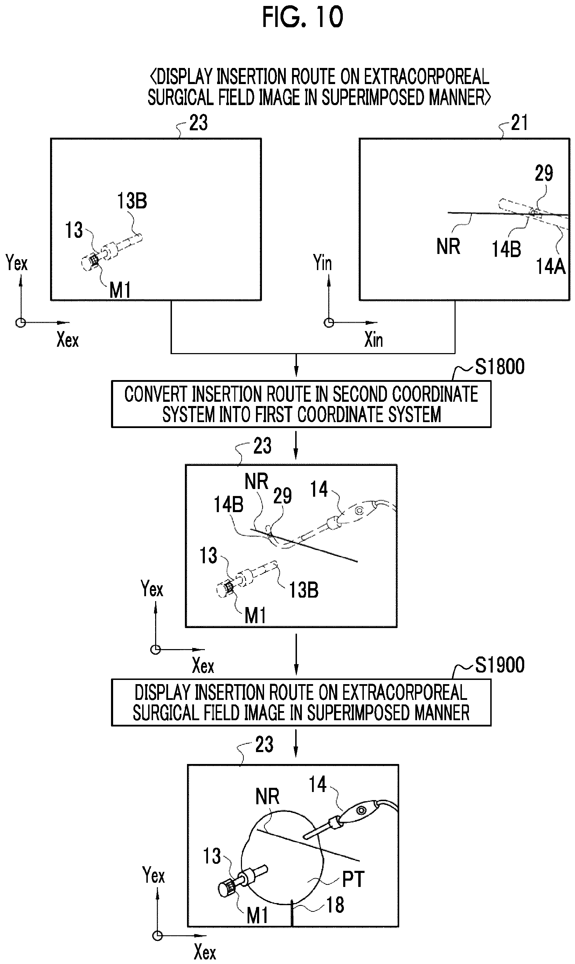

As shown in Fig. 10, in step S1800, the processor 41 performs coordinate conversion of the insertion route NR. The processor 41 derives a correlation between the first coordinate system of the extracorporeal surgical field image 23 and the second coordinate system of the intracorporeal surgical field image 21 captured by the camera 13C, based on an imaging direction of the camera 13C in the distal end portion 13B of the endoscope 13 in the first coordinate system of the extracorporeal surgical field image 23. The processor 41 converts the position and the posture of the insertion route NR in the intracorporeal surgical field image 21 in the second coordinate system into the first coordinate system based on the correlation between the first coordinate system and the second coordinate system. As a result, the position and the posture of the insertion route NR in the extracorporeal surgical field image 23 in the first coordinate system are specified. Then, in step S1900, the processor 41 displays the insertion route NR converted into the first coordinate system in a superimposed manner on the extracorporeal surgical field image 23 of the first coordinate system. That is, the processor 41 executes control to display the composite image in which the insertion route NR is superimposed on the position specified in the extracorporeal surgical field image 23 on the display 16, based on the position and posture information of the distal end portion 13B and the position and posture information of the guide groove 29. The processor 41 repeatedly executes the above processing until a display end instruction is input, such as end of activation of the medical support device 11.

Since the extracorporeal surgical field image 23 is output as a video image, the position and the posture of the insertion route NR in the extracorporeal surgical field image 23 displayed on the display 16 are also updated in accordance with the positions of the endoscope 13, the ultrasound probe 14, the extracorporeal camera 15 and the like in the extracorporeal surgical field SFex, which is three-dimensional real space.

In a case of puncturing the tumor 27 with the puncture needle 18, as shown in Fig. 3, the medical staff ST adjusts the position and the posture of the ultrasound transducer 14C provided at the distal end portion 14B of the ultrasound probe 14 while observing the ultrasound image 22. Specifically, the position and the posture of the ultrasound transducer 14C are adjusted to a position where the puncture line 30 that is an extension line of the guide groove 29 passes through the tumor 27. The position and the posture of the insertion route NR, which are displayed in a superimposed manner on the extracorporeal surgical field image 23, are changed in accordance with the change in the position and the posture of the ultrasound probe 14. In a case where the puncture line 30 is displayed to pass through the tumor 27 in the ultrasound image 22, the puncture line 30 indicates an ideal insertion route of the puncture needle 18. In this state, the position and the posture of the ultrasound probe 14 are fixed. Then, the insertion route NR in the extracorporeal surgical field image 23 in this state also indicates the ideal insertion route of the puncture needle 18.

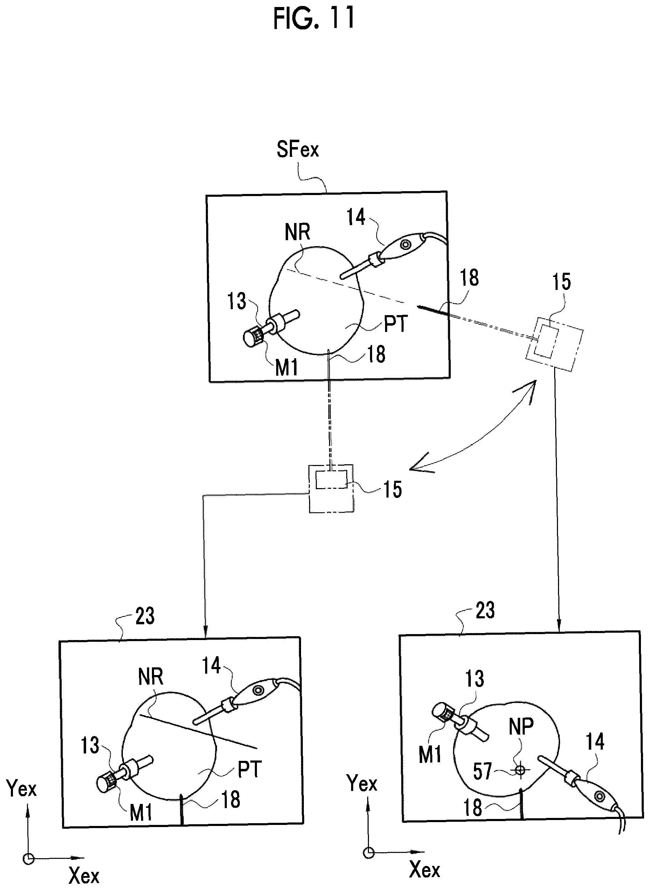

As shown in Fig. 11, while observing the extracorporeal surgical field image 23 in which the ideal insertion route NR is displayed in a superimposed manner, the medical staff ST adjusts the position and the posture of the puncture needle 18 in the extracorporeal surgical field SFex such that the puncture needle 18 is along the ideal insertion route NR.

In the present example, the extracorporeal camera 15 is provided at a proximal end portion of the puncture needle 18. Therefore, since the viewpoint of the extracorporeal camera 15 is also changed in accordance with the position and the posture of the puncture needle 18, the position and the posture of the puncture needle 18 is easily adjusted in accordance with the insertion route NR displayed in the extracorporeal surgical field image 23.

Further, in the present example, the imaging optical axis of the extracorporeal camera 15 is disposed along the axial direction of the puncture needle 18. In this case, in a case where the position and the posture of the puncture needle 18 are adjusted to match the insertion route NR and the axial direction of the puncture needle 18, the viewpoint of the extracorporeal camera 15 at the proximal end of the puncture needle 18 is also changed. As a result, depending on the position and the posture of the puncture needle 18, the insertion route NR, which is displayed as a line in the extracorporeal surgical field image 23 on the lower left side of Fig. 11, may be displayed as a point as shown in the extracorporeal surgical field image 23 on the lower right side of Fig. 11. In a case where the insertion route NR is displayed as the point, the point indicates an insertion position NP on the body surface of the patient PT. In a case where the insertion route NR is displayed as the point, the processor 41 displays the insertion position NP by a mark 57 in which a circular shape and a cross shape are combined, as shown in Fig. 11 as an example. In a case where the insertion position NP is displayed, as long as the puncture needle 18 is inserted toward the insertion position NP indicated by the mark 57 in a state in which the position and the posture of the puncture needle 18 are maintained, the medical staff ST can puncture the tumor 27, which is the target position, with the needle tip of the puncture needle 18 through the guide groove 29 of the ultrasound probe 14. The form of the mark 57 is an example, and of course, other forms may be used.

As described above, the medical support device 11 according to the technique of the present disclosure comprises the processor 41, in which the processor 41 acquires the extracorporeal surgical field image 23 (an example of the captured image) that is captured by the extracorporeal camera 15 provided outside the body of the patient PT (an example of the subject), in which the endoscope 13 (an example of the medical device) whose insertion portion 13 is inserted into the body of the patient PT and the image-recognizable marker M1 which is provided at the grip portion 13D (an example of the portion excluding the insertion portion 13A) of the endoscope 13 are included in the imaging range. Then, the processor 41 derives the position and posture information including at least one of the position or the posture of the distal end portion 13B of the endoscope 13 in the extracorporeal surgical field image 23 based on the marker M1. That is, the processor 41 executes control to display the extracorporeal surgical field image 23 (an example of the composite image) in which the insertion support information (an example of the medical support information) is superimposed on a position specified in the extracorporeal surgical field image 23 on the display 16 (an example of the display unit) based on the position and posture information. Therefore, the medical support device 11 can display the medical support information at an appropriate position corresponding to the position and the posture of a medical device in a simple configuration and a higher degree of freedom of display.

More specifically, the medical support device 11 according to the technique of the present disclosure can provide the medical support information with a simple configuration without using a large-scale device, such as a magnetic navigation device in the related art, by using the marker M1. In addition, the type of medical support information is not limited as in a laser pointer, it is possible to provide information with a higher degree of freedom.

In addition, in the above-described embodiment, the medical device has been described as an example of the endoscope 13 used in the endoscopic surgery. The endoscope 13 has the insertion portion 13A to be inserted into the body from a hole formed in the body surface of the patient PT on a distal end side, and has the marker M1 provided on a proximal end side that is not inserted into the body. The processor 41 derives the position and posture information of the insertion portion 13A in the body based on the marker M1. In a case where the medical device is used for the endoscopic surgery as in the endoscope 13, the position of the insertion portion 13A to be inserted into the body is difficult to recognize from outside the body. Therefore, the technique of the present disclosure is particularly effective. As the medical device used for the endoscopic surgery, in addition to the rigid endoscope shown in the above-described embodiment, a soft endoscope in which the insertion portion 13A is flexible may be used. In addition to the laparoscope, there is also a thoracoscope, an arthroscope, and the like as the endoscope, depending on classifications based on the site to be observed, and the technique of the present disclosure can also be applied to such medical device.

In addition, in the above-described embodiment, the processor 41 derives the position and posture information of the insertion portion 13A based on the marker M1 and the dimensional information of the endoscope 13 (an example of the medical device). By using the dimensional information, it is easy to estimate the position and the posture of the insertion portion 13A inside the body from the marker M1 provided outside the body.

In addition, in the above-described embodiment, the endoscope 13 is provided with the marker M1 (an example of the first marker), the insertion portion 13A (an example of the first insertion portion), and the camera 13C (an example of the intracorporeal camera), and such endoscope 13 is used as an example of the first medical device. Further, as an example of the second medical device different from the endoscope 13, the ultrasound probe 14 provided with the insertion portion 14A (an example of the second insertion portion) to be inserted into the body and the marker M2 (an example of the second marker) is used for surgery together with the endoscope 13. In this case, the processor 41 executes the following processing. That is, the processor 41 acquires the intracorporeal surgical field image 21 (an example of the second captured image) captured by the camera 13C, in which the insertion portion 14A and the marker M2 are included in the imaging range. Then, the position and posture information including at least one of the position or the posture of the insertion portion 14A in the intracorporeal surgical field image 21 is derived based on the marker M2. That is, the processor 41 executes control to display, on the display unit, the composite image in which insertion support information (an example of the medical support information) is superimposed on the extracorporeal surgical field image 23 at a position specified in the extracorporeal surgical field image 23 based on the position and posture information of the distal end portion 13B of the insertion portion 13A (an example of first position and posture information) and the position and posture information of the guide groove 29 of the insertion portion 14A (an example of second position and posture information).

In this way, even in a case where two medical devices of the first medical device and the second medical device, such as the endoscope 13 and the ultrasound probe 14, are used, it is possible to grasp a relative positional relationship of the plurality of medical devices (see relative positional relationships RP1-1, RP1-2, RP1-3, and RP2 in Fig. 3) by using the two markers of the marker M1 and the marker M2. Accordingly, it is possible to display the medical support information at an appropriate position corresponding to the position and the posture of each medical device.

In the above-described embodiment, the first medical device is the endoscope 13, and the second medical device is the ultrasound probe 14 (an example of a medical probe) capable of observing an internal structure of an organ such as the liver LV. Since both the endoscope 13 and the ultrasound probe 14 have an insertion portion to be inserted into the body, the technique of the present disclosure for estimating information inside the body from information outside the body is particularly effective. In addition, as the medical probe, optical coherence tomography (OCT) or the like may be used instead of the ultrasound probe 14.

In the above-described embodiment, the medical support information is insertion support information of the puncture needle 18 (an example of the treatment tool) to be inserted from the outside the body toward a position of the tumor 27 (an example of the target position) in the liver LV (an example of the organ) observed through the ultrasound probe 14 (an example of the medical probe), and is insertion support information including at least one of the insertion position NP or the insertion route NR. Accordingly, the insertion of the treatment tool can be performed more accurately.

In addition, the ultrasound probe 14 is relatively often used in combination with the treatment tool such as the puncture needle 18. Therefore, as in the embodiment described above, in a case where the ultrasound probe 14 is used as the medical probe, the technique of the present disclosure is particularly effective. In addition, the puncture needle 18 is often required to accurately puncture the target position. Therefore, the technique of the present disclosure of providing the medical support information, such as the insertion support information, is particularly effective in a case where the puncture needle 18 is used as the treatment tool.

In addition, in the above-described embodiment, as shown in Fig. 11, the insertion position NP is displayed by a mark 57 indicating a position on the body surface of the patient PT into which the puncture needle 18 is inserted. Therefore, even from the outside the body, the insertion position of the puncture needle 18 can be easily confirmed, and the puncture needle 18 can be accurately inserted as compared with a case where the insertion position of the puncture needle 18 is not displayed by the mark 57. Further, in the above-described embodiment, since the insertion route NR is indicated by a line, the puncture needle 18 can be accurately inserted as compared with a case where the insertion route NR is not displayed by the line.

In addition, in the above-described embodiment, the ultrasound probe 14 that is an example of the medical probe is provided in the insertion portion 14A and has the guide groove 29 for guiding the insertion of the puncture needle 18 to the target position by engaging with the puncture needle 18 (an example of the treatment tool). The processor 41 specifies a position at which the insertion support information is superimposed on the extracorporeal surgical field image 23 based on a relative positional relationship between the marker M2 and the guide groove 29. As an example, the dimensional information of the ultrasound probe 14 is used as the relative positional relationship. Therefore, it is possible to specify the position on which the insertion support information is superimposed with relatively simple processing as compared with a case where the relative positional relationship is not used.

Further, in the above-described embodiment, the extracorporeal camera 15 is provided on the proximal end side of the puncture needle 18 (an example of the treatment tool) and is capable of outputting the extracorporeal surgical field image 23 as a video image, and the processor 41 displays a line indicating the insertion route NR in the video image as the insertion support information. Therefore, since the viewpoint of the extracorporeal camera 15 is also changed in accordance with the position and the posture of the puncture needle 18, the position and the posture of the puncture needle 18 is easily adjusted in accordance with the insertion route NR displayed in the extracorporeal surgical field image 23.

Further, the axial direction of the puncture needle 18 and the imaging optical axis of the extracorporeal camera 15 match each other. Therefore, as shown in Fig. 11, the position and the posture of the puncture needle 18 are adjusted such that the insertion route NR displayed in the extracorporeal surgical field image 23 captured by the extracorporeal camera 15 matches the axial direction of the puncture needle 18, so that the line of the insertion route NR is displayed as a point indicating the insertion position NP. Therefore, the insertion position NP of the puncture needle 18 on the body surface can be accurately displayed with a simple configuration.

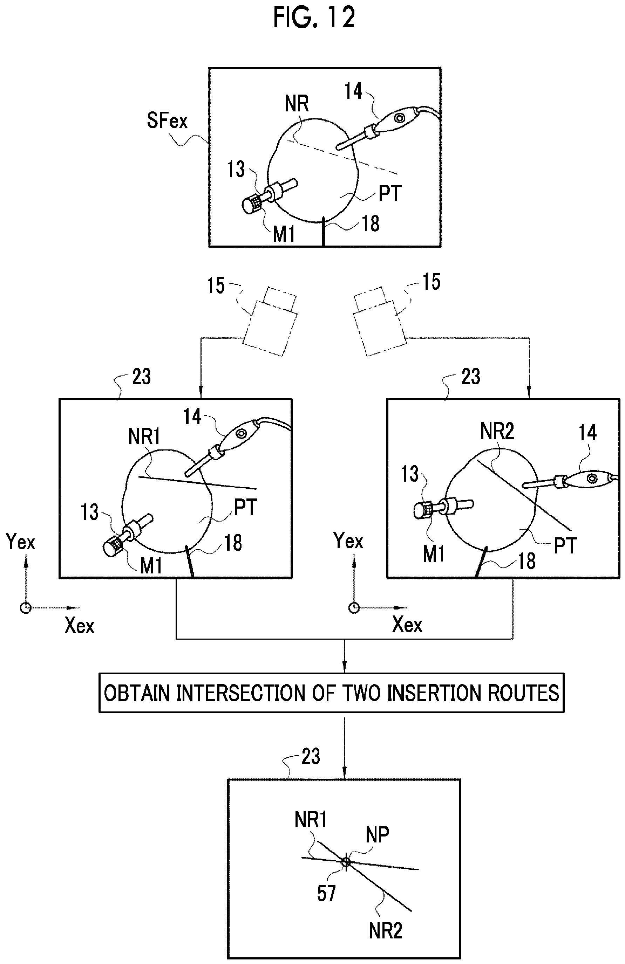

The effect of deriving the insertion position NP on the body surface of the patient PT as shown in Fig. 11 can also be obtained by the method shown in Fig. 12. That is, as shown in Fig. 12, two or more extracorporeal surgical field images 23 having different viewpoints are acquired by changing the viewpoint of the extracorporeal camera 15, and the insertion route NR is displayed on each of the acquired images. The insertion route NR of the extracorporeal surgical field image 23 of one viewpoint is set as NR1, and the insertion route NR of the other viewpoint is set as NR2. The processor 41 obtains an intersection of the two insertion routes NR1 and NR2 shown in the two extracorporeal surgical field images 23 to derive the intersection as an insertion position NP on the body surface of the patient PT. The method shown in Fig. 12 is, in short, a method of deriving the insertion position NP in the manner of stereo imaging using parallax.

In the example shown in Fig. 12, the extracorporeal surgical field images 23 having different viewpoints are acquired by moving one extracorporeal camera 15, but two or more extracorporeal cameras 15 having different viewpoints may be used.

In addition, in the above-described embodiment, as a method of specifying a position at which the insertion support information, such as the insertion route NR, is superimposed on the extracorporeal surgical field image 23, the method shown in Fig. 10 is used. That is, the insertion route NR in the intracorporeal surgical field image 21 of the second coordinate system captured by the camera 13C of the endoscope 13 is converted into the first coordinate system of the extracorporeal surgical field image 23 to specify a position to be superimposed on the extracorporeal surgical field image 23.

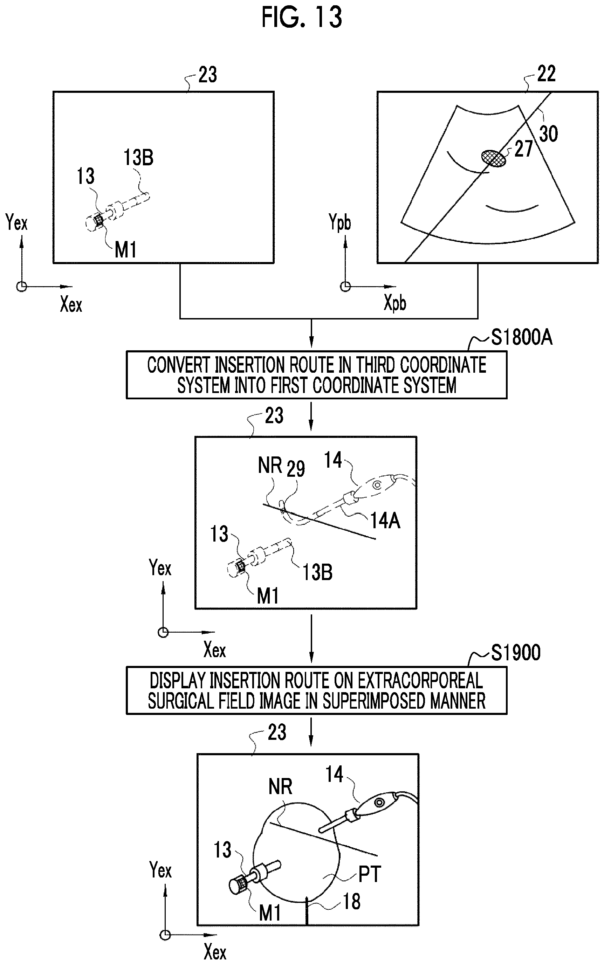

In addition to such a specifying method, the method shown in Fig. 13 may be used. First, as shown in Fig. 3, in the ultrasound image 22 acquired by the ultrasound probe 14, the tumor 27 as the target position is detected, and a case where the position and the posture of the ultrasound probe 14 are in an ideal state in which the puncture line 30 passes through the tumor 27 is considered. The method shown in Fig. 13 is a method of converting a puncture line 30 in a third coordinate system in the ultrasound image 22 into the first coordinate system of the extracorporeal surgical field image 23 in a case where the coordinate system of the ultrasound image 22 is the third coordinate system. As shown in Fig. 10, by using the marker M2, it is possible to grasp the correlation between the first coordinate system of the extracorporeal surgical field image 23 and the second coordinate system of the intracorporeal surgical field image 21 in which the distal end portion 14B of the ultrasound probe 14 is shown. In addition, in a case where the position and the posture of the distal end portion 14B can be estimated, it is also possible to estimate the position and the posture of the ultrasound transducer 14C of the distal end portion 14B from the dimensional information. In this way, it is possible to grasp a correlation between the second coordinate system of the intracorporeal surgical field image 21 in which the distal end portion 14B of the ultrasound probe 14 is shown and the third coordinate system of the ultrasound image 22 acquired by the ultrasound probe 14.

That is, the processor 41 specifies the position at which the insertion support information is superimposed on the extracorporeal surgical field image 23 based on a correlation between the third coordinate system of the ultrasound image 22, which is derived based on the tumor 27 (an example of the target position) specified in the ultrasound image 22 (an example of the internal image) of the liver LV (an example of the organ) acquired by the ultrasound probe 14 (an example of the medical probe) and the position and posture information of the distal end portion 14B, and the first coordinate system of the extracorporeal surgical field image 23. According to this method, it is possible to effectively use the internal image acquired by the medical probe, such as the ultrasound probe 14, for specifying the position on which the insertion support information is superimposed.

### Second embodiment

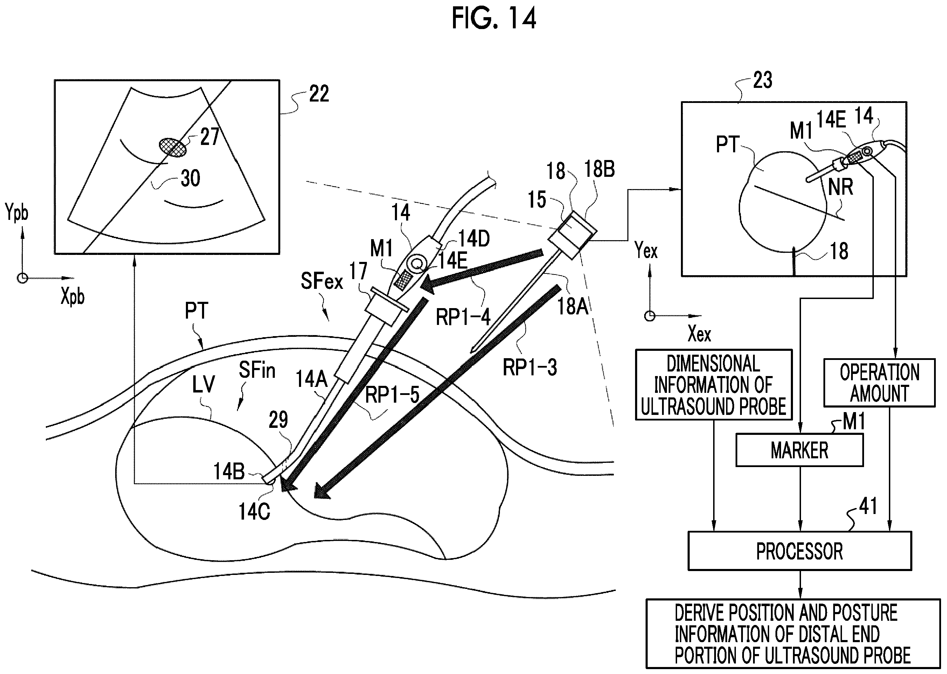

In the above-described embodiment, an example has been described in which the endoscope 13 is used as the first medical device having the marker M1 that is the first marker, and the ultrasound probe 14 is used as the second medical device having the marker M2 that is the second marker. However, the number of medical devices having the marker may be only one. A second embodiment shown in Fig. 14 is an example of a medical device having a marker. In Fig. 14, the medical device having a marker is an ultrasound probe 14 capable of observing an internal structure of an organ. The ultrasound probe 14 is provided with a marker M1. The marker M1 is provided in an operation portion 14D on a proximal end side, which is a portion excluding an insertion portion 14A, in the ultrasound probe 14.

An extracorporeal camera 15 images an extracorporeal surgical field SFex such that the marker M1 of the operation portion 14D is included in an imaging range in a state in which the insertion portion 14A of the ultrasound probe 14 is inserted into a body of a patient PT. A processor 41 acquires an extracorporeal surgical field image 23 captured by the extracorporeal camera 15. The processor 41 derives a position and a posture of a guide groove 29 provided in a distal end portion 14B of the ultrasound probe 14 inserted into the body in the same manner as in the derivation of the position and posture information of the distal end portion 13B of the endoscope 13 in the first embodiment.

That is, the processor 41 estimates the position and the posture of the guide groove 29 of the ultrasound probe 14 based on the marker M1 detected from the extracorporeal surgical field image 23 and dimensional information of the ultrasound probe 14, and derives position and posture information of the distal end portion 14B. Then, the processor 41 estimates the position and the posture of the guide groove 29 of the distal end portion 14B based on the dimensional information. The processor 41 specifies a position on which an insertion route NR is superimposed in accordance with the position and the posture of the guide groove 29 in the extracorporeal surgical field image 23, and generates a composite image in which the insertion route NR is superimposed on the specified position.

As described above, the second embodiment is conceptually a method of grasping a relative positional relationship RP1-3 between the extracorporeal camera 15 and the distal end portion 14B based on a relative positional relationship RP1-4 between the extracorporeal camera 15 and the marker M1 of the ultrasound probe 14, and a positional relationship RP1-5 between the marker M1, which is defined by the known dimensional information, and the distal end portion 14B.

In addition, in a case where the distal end portion 14B to be inserted into the body, such as the ultrasound probe 14, is bendable, it is preferable to consider an operation amount of the bending operation of the distal end portion 14B. As shown in Fig. 14, the extracorporeal camera 15 captures the extracorporeal surgical field image 23 such that an angle knob 14E provided in the operation portion 14D of the ultrasound probe 14 is included in the imaging range, together with the marker M1. The angle knob 14E is an operation portion that changes a position and a posture of the distal end portion 14B by bending the distal end portion 14B, for example, by a rotation operation.

The processor 41 detects a rotation amount of the angle knob 14E as an operation amount by performing image analysis on the extracorporeal surgical field image 23. For example, a detection marker for detecting the rotation amount is provided in the angle knob 14E, and the processor 41 detects the operation amount by performing image recognition on a change and an amount of the changes of the position and the posture of the detection marker. The rotation amount also includes a rotation direction.

The processor 41 corrects, based on the operation amount, the position and the posture of the distal end portion 14B estimated based on the dimensional information to estimate the position and the posture of the distal end portion 14B in accordance with the operation amount.

As described above, in a case where the medical device having the marker M1 is capable of changing a position and a posture of the insertion portion by operating the operation portion (such as the angle knob 14E) provided outside the body as in the ultrasound probe 14 according to the second embodiment, the processor 41 acquires an operation amount of the operation portion in addition to the dimensional information, and uses the acquired operation amount to derive position and posture information the insertion portion. Accordingly, it is possible to more accurately estimate the position and the posture of the insertion portion.

The detection of the operation amount of the operation portion may be performed using an electrical signal instead of the detection marker. For example, a pulse signal having the number of pulses corresponding to the rotation amount is transmitted from the ultrasound probe 14 to the processor 41 in response to the rotation operation of the angle knob 14E. The processor 41 can detect the rotation amount of the angle knob 14E based on the pulse signal. The transmission of the pulse signal may be performed in a wireless manner or in a wired manner.

### Other Modification Examples

Each of the above-described embodiments is an example, and various modifications can be made to the technique of the present disclosure. In the above-described embodiment, the endoscopic surgery has been described as an example, but it can also be applied to laparotomy.

### Example of laparotomy

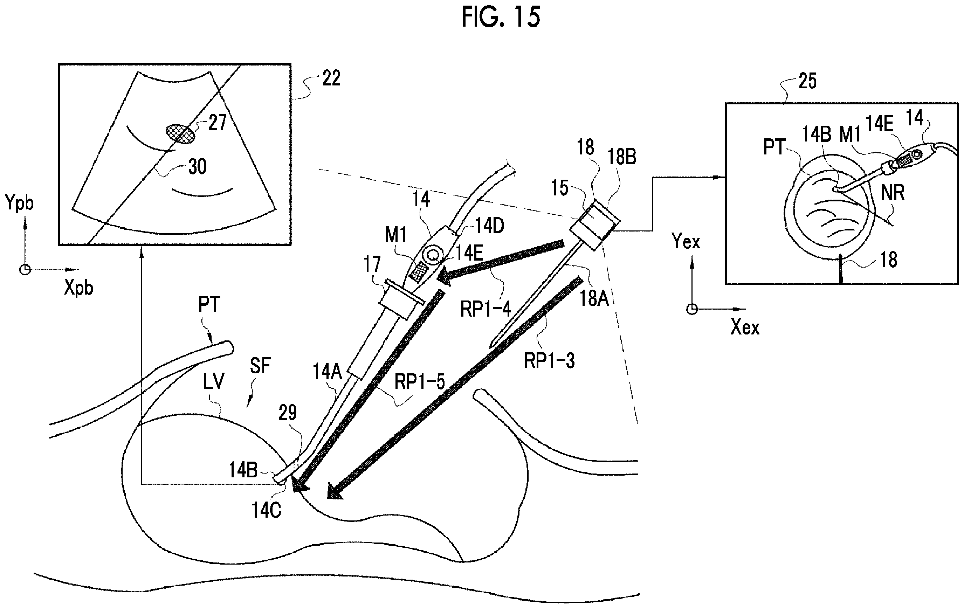

The example shown in Fig. 15 is an example applied to laparotomy. Although there is a difference between the laparotomy and the endoscopic surgery, even in the example shown in Fig. 15, the basic configuration is the same as that in Fig. 14, and thus only the difference will be described. In a case of laparotomy, since a boundary between the extracorporeal surgical field and the intracorporeal surgical field is not clear, the extracorporeal camera 15 captures a surgical field image 25 of the surgical field SF in which intracoporeal surgical field and the extracorporeal surgical field are mixed. The marker M1 is included in an imaging range of the surgical field image 25. The processor 41 derives the position and posture information of the distal end portion 14B of the ultrasound probe 14 based on the surgical field image 25. Then, in the surgical field image 25, a composite image in which the insertion support information such as the insertion route NR is superimposed on the position specified based on the derived position and posture information is generated.

### Example of use for robot surgery

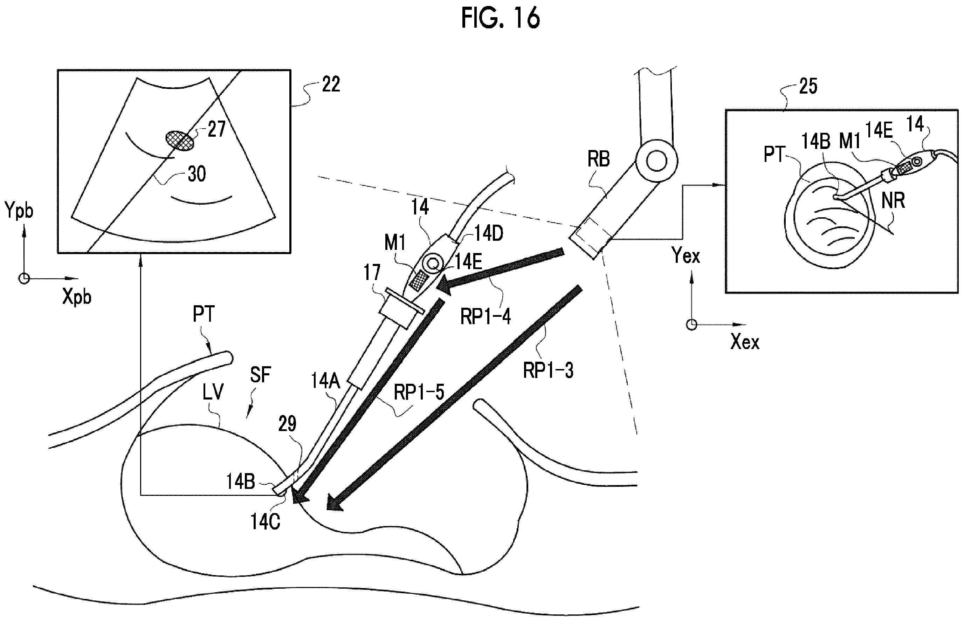

In addition, as shown in Fig. 16, the technique of the present disclosure can also be applied to surgery using a robot RB. The robot RB has, for example, a displaceable arm, and an extracorporeal camera 15 is provided at a distal end portion of the arm. The extracorporeal camera 15 images a marker M1 of an ultrasound probe 14. The processor 41 derives position and posture information of the distal end portion 14B based on the marker M1 of the ultrasound probe 14 and generates a composite image in which the insertion support information such as the insertion route NR is superimposed on the position specified based on the derived position and posture information in the surgical field image 25.

In the example shown in Fig. 16, the example in which the extracorporeal camera 15 is provided in the robot RB has been described, but the present disclosure is not limited to this, and various uses cam be made. For example, the robot RB may be a medical device having the marker M1, such as the ultrasound probe 14. In this case, a part of the arm of the robot RB is an insertion portion to be inserted into the body, and the marker M1 is provided in a portion excluding the insertion portion. Then, the marker M1 is imaged by the extracorporeal camera 15 provided independently of the robot RB. The processor 41 detects the marker M1 from the surgical field image 25, and estimates the position and the posture of the insertion portion of the robot RB based on the detected marker M1. The processor 41 generates a composite image in which insertion support information is superimposed on the surgical field image 25 at a position specified based on the position and posture information.

### Third embodiment

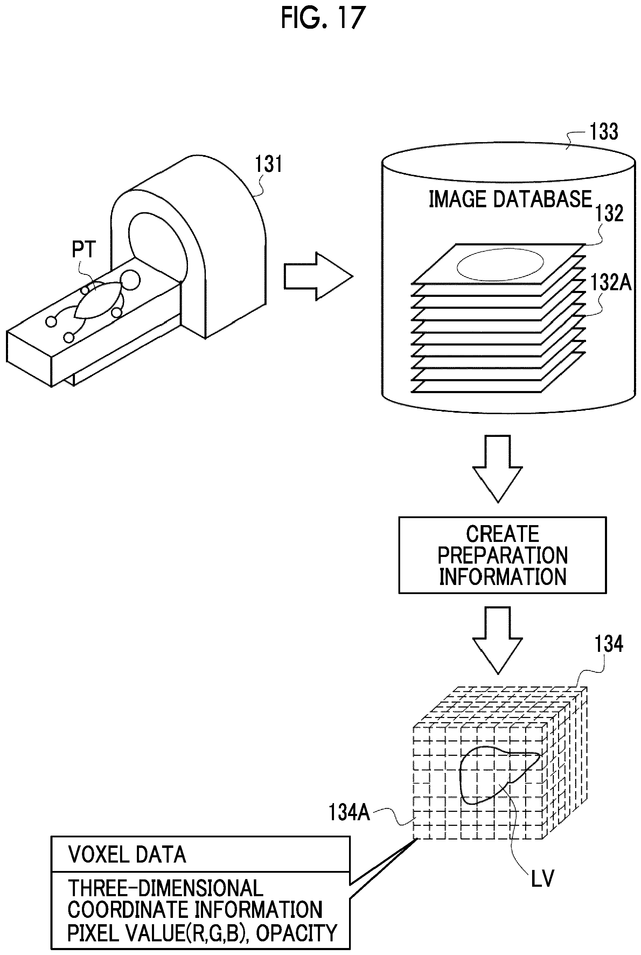

In the above-described embodiment, an example in which the insertion support information, such as the insertion route NR to be superimposed on the extracorporeal surgical field image 23, is generated in the ultrasound image 22 or the intracorporeal surgical field image 21, and is converted into the coordinate system of the extracorporeal surgical field image 23. However, as shown in the third embodiment shown in Figs. 17 to 20, the method of generating the insertion support information may be other methods. The insertion support information may be prepared in advance before surgery, and the prepared insertion support information may be superimposed on the extracorporeal surgical field image 23.

### Creation of presurgical preparation information

In the third embodiment, for example, before the surgery, presurgical simulation is performed using three-dimensional (3D) data of a virtual endoscope, and the insertion support information, such as the insertion route NR of a puncture needle 18, is prepared in the presurgical simulation. In addition, the third embodiment is an example in which the endoscope 13 is used as the first medical device and the ultrasound probe 14 is used as the second medical device, as in the first embodiment. Hereinafter, the description will be made with reference to Figs. 17 to 20.

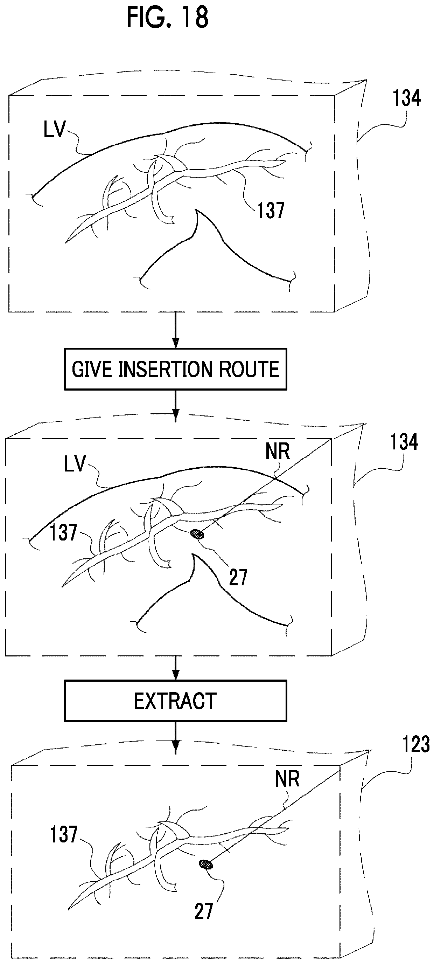

First, with reference to Figs. 17 and 18, a method of creating the insertion route NR of the puncture needle 18 that punctures a liver LV as the insertion support information in the presurgical simulation using the 3D data of the virtual endoscope will be described. In addition, the 3D data of the virtual endoscope is generated based on a tomographic image group 132 captured in advance by a tomography apparatus 131, such as a computed tomography (CT) apparatus and a magnetic resonance imaging (MRI) apparatus. In a case where the tomography apparatus 131 is the CT apparatus, a CT value is acquired while rotating a radiation source and a radiation detector around a body axis of a patient PT. The acquisition of the CT value is performed at each position in a body axis direction by scanning the radiation source and the radiation detector in the body axis direction of the patient PT. The CT value is a radiation absorption value in the body of the patient PT. The tomography apparatus 131 generates the tomographic image 132A by executing image reconstruction processing based on the CT value acquired in each direction around the body axis. Each tomographic image 132A is a two-dimensional image generated depending on a slice thickness in the body axis direction, and the tomographic image group 132 is a set of a plurality of tomographic images 132A corresponding to respective positions in the body axis direction. The tomographic image group 132 is output to an image database 133, such as a picture archiving and communication system (PACS).

An information processing apparatus (not shown) generates a three-dimensional image 134 that is a set of voxel data 134A by performing 3D modeling that numerically describes a three-dimensional shape of the body of the patient PT based on the tomographic image group 132 obtained by the tomography apparatus 131. The voxel data 134A is a unit of a pixel in a three-dimensional space, and has three-dimensional coordinate information and a pixel value. The three-dimensional image 134 generated by the 3D modeling is also referred to as three-dimensional volume data or the like. In the tomographic image group 132, pixel intervals of each tomographic image 132A and slice thicknesses of each tomographic image 132A may be different from each other. In this case, for example, in the 3D modeling, the three-dimensional image 134 having the isotropic voxel data 134A in which the length in each three-dimensional direction are equal to each other is generated by performing the interpolation processing of the adjacent tomographic images 132A. Here, since the three-dimensional image 134 generated based on the tomographic image group 132 is information created before the surgery, the three-dimensional image 134 is referred to as a presurgical 3D image 134 for convenience. The presurgical 3D image 134 is an example of 3D data of the virtual endoscope, and is further an example of a "three-dimensional image of an organ acquired in advance before the surgery" according to the technique of the present disclosure.

The presurgical 3D image 134 is an image capable of reproducing an external shape of the body of the patient PT, an anatomical site such as an organ in the body, and an internal structure thereof. The presurgical 3D images 134 shown in Figs. 17 and 18 show data of the liver LV as an example of the anatomical site. The presurgical 3D image 134 also includes data capable of reproducing a vascular structure which is an example of the internal structure of the liver LV.

In addition, the presurgical 3D image 134 of the present example is a color image, and each of red (R), green (G), and blue (B) is given as the pixel value of the voxel data 134A. The presurgical 3D image 134 may be a monochrome image. For example, the pixel value of the voxel data 134A may be represented by only the brightness (Y) based on the CT value. In addition, a value obtained by converting the CT value by using a preset look up table (LUT) or an arithmetic expression may be used as the pixel value of the voxel data 134A. Further, the pixel value of the voxel data 134A may be set to a color associated with each specific site, such as the organ or a lesion, specified in the presurgical 3D image 134. In addition, the voxel data 134A is also set with an opacity. The opacity is data used in volume rendering. The rendering is processing of converting a part of the presurgical 3D image 134 into a two-dimensional projection image, and the volume rendering is a rendering method that also projects internal information of an object included in the presurgical 3D image 134 onto the projection image. By setting the opacity for each voxel data 134A, in a case where the volume rendering is performed, it is possible to appropriately use representations of the internal information such as projecting to the projection image in an opaque manner, projecting to the projection image in a translucent manner, and projecting to the projection image in a transparent manner.

As shown in Fig. 18, since information 123 including the insertion route NR is information prepared in advance before the surgery, the information 123 is referred to as presurgical preparation information. The presurgical preparation information 123 is generated based on the presurgical 3D image 134. The insertion route NR is given to the presurgical 3D image 134 in the surgery simulation performed before surgery using the presurgical 3D image 134. The presurgical preparation information 123 includes a vascular structure 137 and a tumor 27 as an internal structure of a liver LU to be inserted, in addition to the insertion route NR. The presurgical preparation information 123 including the insertion route NR and the internal structure, such as the vascular structure 137 and the tumor 27, is extracted from the presurgical 3D image 134.

As described above, the presurgical preparation information 123 is information in which the three-dimensional position in the presurgical 3D image 134 is defined. The vascular structure 137 included in the presurgical preparation information 123 is used for registration with the ultrasound image 22 acquired by the ultrasound probe 14.

### Acquisition of ultrasound 3D image

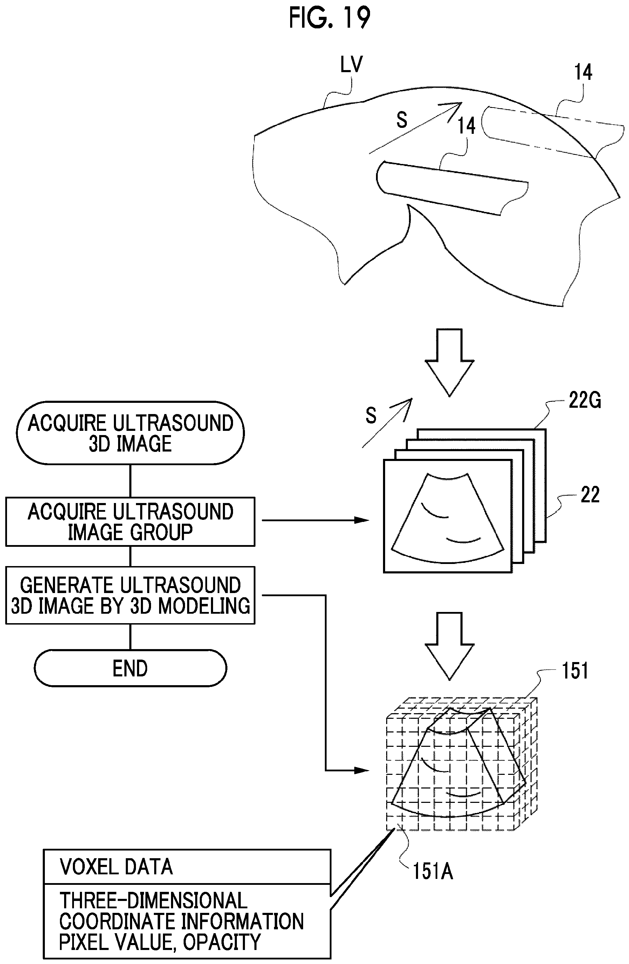

As shown in Fig. 19, the processor 41 acquires an ultrasound three-dimensional (3D) image 151 based on an ultrasound image group 22G which is a set of ultrasound images 22 captured by the ultrasound probe 14. The processor 41 uses the ultrasound 3D image 151 acquired during the surgery for registration. The ultrasound 3D image 151 is an example of an "internal image of an organ acquired by a medical probe" according to the technique of the present disclosure.

As shown in Fig. 19, first, in a case where the ultrasound probe 14 is caused to perform the scan along a surface of a target site, such as a liver LV, a plurality of the ultrasound images 22 at each of the positions on a scanning trajectory S can be acquired. The processor 41 acquires the ultrasound image group 22G and performs 3D modeling based on the acquired ultrasound image group 22G to generate the ultrasound 3D image 151. The ultrasound image 22 is a tomographic image as in the tomographic image 132A shown in Fig. 17, and the ultrasound image group 22G is the same as the tomographic image group 132. In the ultrasound image group 22G, the internal structure of the target site, such as the vascular structure 137 of the liver LV, is also depicted.

The processor 41 generates the ultrasound 3D image 151 by executing the same processing as the 3D modeling described with reference to Fig. 17. The ultrasound 3D image 151 is a set of voxel data 151A, which are pixels in a three-dimensional space, as in the presurgical 3D image 134. The voxel data 151A is also the same as the voxel data 134A shown in Fig. 17, and a three-dimensional coordinate, the pixel value, and the opacity are set.

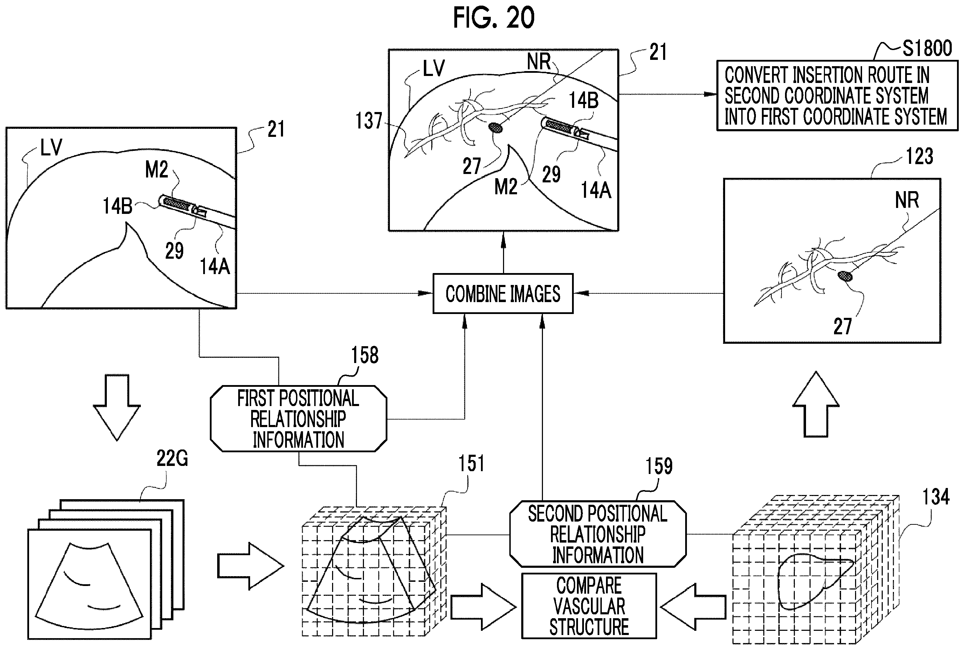

As shown in Fig. 20, the processor 41 derives first positional relationship information 158 that is a correlation between a coordinate system of the intracorporeal surgical field image 21 acquired through the endoscope 13 and a coordinate system of the ultrasound 3D image 151, and second positional relationship information 159 that is a correlation between the coordinate system of the ultrasound 3D image 151 and a coordinate system of the presurgical 3D image 134.

First, a method of deriving the first positional relationship information 158 will be described. The processor 41 estimates the position and the posture of an ultrasound transducer 14C based on a marker M2 of the ultrasound probe 14 shown in the intracorporeal surgical field image 21 in the same manner that described in the first embodiment. Then, the processor 41 estimates a position and a posture of the ultrasound 3D image 151 in the intracorporeal surgical field image 21 based on the estimated position and posture of the ultrasound transducer 14C. The position and the posture of the ultrasound 3D image 151 in the intracorporeal surgical field image 21 are the first positional relationship information 158. The first positional relationship information 158 is an example of a "correlation between a coordinate system of an internal image and a coordinate system of a second captured image that is derived based on second position and posture information" according to the technique of the present disclosure.

Next, a method of deriving the second positional relationship information 159 will be described. The processor 41 compares the vascular structures 137 depicted in each of the ultrasound 3D image 151 and the presurgical 3D image 134. Specifically, similar structures of both vascular structures 137 are searched, and registration of the ultrasound 3D image 151 and the presurgical 3D image 134 is performed such that both similar structures are matched with each other. The search for the similar structures of the vascular structures 137 may be performed using a rule-based image analysis method such as pattern matching or a method using an artificial intelligence technique such as semantic segmentation. The processor 41 can derive the second positional relationship information 159 by performing the registration using the vascular structure 137.

Then, the processor 41 combines the presurgical preparation information 123 including the insertion route NR, which is generated based on the presurgical 3D image 134, and the intracorporeal surgical field image 21, based on the first positional relationship information 158 and the second positional relationship information 159. Since registration is performed by the vascular structure 137 of the liver LV, the insertion route NR is superimposed on an appropriate position with respect to the tumor 27. The processor 41 generates a composite image in which the insertion route NR in the intracorporeal surgical field image 21 in a second coordinate system is superimposed on the extracorporeal surgical field image 23 in a first coordinate system by performing coordinate conversion described in step S1800 (see Figs. 5 and 10) of the first embodiment. The second positional relationship information 159 is an example of a "correlation between a coordinate system of an internal image of the organ acquired by the medical probe and a coordinate system of a three-dimensional image" according to the technique of the present disclosure.

As described above, in a case where the insertion route NR (an example of the insertion support information) is set by the simulation before the surgery in the presurgical 3D image 134 (an example of the three-dimensional image of the organ) acquired in advance before the surgery, the processor 41 superimposes the insertion route NR on the extracorporeal surgical field image 23 by using the second positional relationship information 159 and the first positional relationship information 158. As a result, it is possible to effectively use the internal image of the medical probe and the result of the presurgical simulation. In the presurgical simulation, for example, it is possible to set the appropriate insertion route NR that is unlikely to damage the vascular structure 137. By providing the insertion route NR in the extracorporeal surgical field image 23, it is possible to perform more appropriate medical support.

In the example of Fig. 20, in addition to the insertion route NR, information on the vascular structure 137 and the tumor 27 is also displayed in a superimposed manner on the intracorporeal surgical field image 21. In Fig. 20, the information on the vascular structure 137 or the like is displayed in a superimposed manner on the intracorporeal surgical field image 21, but may be superimposed and displayed on the extracorporeal surgical field image 23.

In addition, in each of the above-described embodiments, cauterization has been described as an example of the function of the puncture needle, but the function of the puncture needle may be a function other than cauterization. Further, although the puncture needle has been described as an example of the treatment tool, in addition to the puncture needle, a treatment tool for injecting a fluorescent agent such as indocyanine green (ICG), a biopsy needle used for collecting a tissue for performing a biopsy, a forceps, or the like may be used.

In addition, in each of the above embodiments, the insertion support information, such as the insertion position NP and the insertion route NR of the puncture needle has been exemplified as the medical support information, but the medical support information may be information indicating a distal end position, a shape, posture information, or the like of the medical device inserted into the body.

In addition, in each of the above-described embodiments, the case where the extracorporeal camera 15 is provided in the puncture needle 18 or the robot RB has been described, but the installation position of the extracorporeal camera 15 is not limited to the above. For example, extracorporeal camera 15 may be set at a position where the extracorporeal surgical field SFex can be imaged, such as a ceiling of the operating room.

In addition, in each of the above-described embodiments, the inside of the abdominal cavity has been described as an example of the inside of the body, but the inside of the body is not limited to the inside of the abdominal cavity, and may be a body cavity other than the abdominal cavity such as a thoracic cavity, or may be an inside of a tubular organ such as an upper gastrointestinal tract such as the esophagus, a lower gastrointestinal track such as the intestine, or a bronchus. In a case where the technique of the present disclosure is applied to a surgical field in the tubular organ, for example, a marker M1 is provided in a proximal end portion of a soft endoscope to be inserted into the tubular organ.

In each of the above-described embodiments, the medical device having the insertion portion may be a trocar 17, in addition to the endoscope 13 and the medical probe (the ultrasound probe 14 or an OCT probe).

In the above-described embodiment, for example, as a hardware structure of processor 41 that executes various kinds of processing, such as an acquisition of an image, a derivation of position and posture information, a generation of a composite image, and a display control, various processors described below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific processing, such as an application specific integrated circuit (ASIC), in addition to a CPU that is a general-purpose processor configured to execute software (program) to function as various processing units.

Various kinds of processing described above may be executed by one of various processors or may be executed by a combination of two or more processors (for example, a combination of a plurality of FPGAs or a CPU and an FPGA) of the same type or different types. A plurality of processing units may be configured with one processor. As an example where a plurality of processing units are configured with one processor, there is a form in which a processor that realizes all functions of a system including a plurality of processing units into one integrated circuit (IC) chip is used, such as system on chip (SOC).

In this way, various processing units are configured using one or more processors among various processors described above as a hardware structure.

In addition, as the hardware structure of various processors, more specifically, an electric circuit (circuitry), in which circuit elements, such as semiconductor elements, are combined can be used.

In addition to the operation program of a medical support device, the technique of the present disclosure extends to a computer readable storage medium (a USB memory or a digital versatile disc (DVD)-read only memory (ROM), or the like) that stores the operation program of a medical support device in a non-transitory manner.

The content of the above description and the content of the drawings are detailed description of portions according to the technique of the present disclosure, and are merely examples of the technique of the present disclosure. For example, the above description relating to configuration, function, operation, and advantageous effects is description relating to configuration, function, operation, and advantageous effects of the portions according to the technique of the present disclosure. Thus, it is needless to say that unnecessary portions may be deleted, new elements may be added, or replacement may be made to the content of the above description and the content of the drawings without departing from the gist of the technique of the present disclosure. Furthermore, to avoid confusion and to facilitate understanding of the portions according to the technique of the present disclosure, description relating to common technical knowledge and the like that does not require particular description to enable implementation of the technique of the present disclosure is omitted from the content of the above description and the content of the drawings.

In the specification, "A and/or B" is synonymous with "at least one of A or B". That is, "A and/or B" may refer to A alone, B alone, or a combination of A and B. Furthermore, in the specification, a similar concept to "A and/or B" applies to a case in which three or more matters are expressed by linking the matters with "and/or".

All cited documents, patent applications, and technical standards described in the specification are incorporated by reference in the specification to the same extent as in a case where each individual cited document, patent application, or technical standard is specifically and individually indicated to be incorporated by reference.

## Claims

1. A medical support device (11) comprising:
a processor (41),
wherein the processor (41) is configured to:
acquire a captured image (23) that is captured by an extracorporeal camera (15) provided outside a body of a subject and in which a medical device (13) whose insertion portion (13A) is inserted into the body of the subject and a marker (M1) which is provided at a portion of the medical device excluding the insertion portion and is image-recognizable are included in an imaging range;
derive position and posture information including at least one of a position or a posture of the medical device in the captured image (23) based on the marker (M1); and
execute a control of displaying, on a display unit (16), a composite image in which medical support information is superimposed at a position specified in the captured image (23) based on the position and posture information.

2. The medical support device according to claim 1,
wherein the medical device (13) is used for an endoscopic surgery, has an insertion portion (13A) to be inserted into the body from a hole formed in a body surface of the subject on a distal end side, and has the marker provided on a proximal end side that is not inserted into the body, and
the processor (41) is configured to derive the position and posture information of the insertion portion of the medical device based on the marker.

3. The medical support device according to claim 2,
wherein the processor (41) is configured to derive the position and posture information of the insertion portion based on the marker and dimensional information of the medical device.

4. The medical support device according to claim 3,
wherein, in a case where the medical device (13) is configured to change a position and a posture of the insertion portion (13A) by operating an operation portion provided outside the body,
the processor (41) is configured to acquire an operation amount of the operation portion in addition to the dimensional information, and
the acquired operation amount is used for deriving the position and posture information of the insertion portion (13A).

5. The medical support device according to claim 2,
wherein the medical device is a first medical device having an intracorporeal camera (13C) for imaging the inside of the body, and
in a case where the insertion portion (13A) of the first medical device (13) is set as a first insertion portion, the marker of the first medical device is set as a first marker (M1), the captured image is set as a first captured image (23), and the position and posture information is set as first position and posture information, and
further, a second medical device (14), which is different from the first medical device and is provided with a second insertion portion (14A) to be inserted into the body and a second marker (M2) that is image-recognizable in the second insertion portion, is used for surgery together with the first medical device,
the processor is configured to:
acquire a second captured image (21) which is captured by the intracorporeal camera (13C) and in which the second insertion portion (14A) and the second marker (M2) are included in an imaging range;
derive second position and posture information including at least one of a position or a posture of the second insertion portion (14A) in the second captured image (21) based on the second marker; and
execute a control of displaying, on the display unit (16), a composite image in which the medical support information is superimposed on the first captured image at a position specified in the first captured image based on the first position and posture information and the second position and posture information.

6. The medical support device according to claim 2,
wherein the medical device is a medical probe that is configured to observe an internal structure of an organ.

7. The medical support device according to claim 5,
wherein the first medical device (13) is an endoscope, and
the second medical device (14) is a medical probe that is configured to observe an internal structure of an organ.

8. The medical support device according to claim 6 or 7,
wherein the medical support information is insertion support information of a treatment tool (18) that is inserted from outside the body toward a target position in the organ observed through the medical probe, and is the insertion support information including at least one of an insertion position or an insertion route.

9. The medical support device according to claim 8,
wherein the medical probe is an ultrasound probe.

10. The medical support device according to claim 8,
wherein the treatment tool (18) is a puncture needle.

11. The medical support device according to claim 10,
wherein the insertion position is displayed by a mark indicating a position on the body surface of the subject at which the puncture needle is inserted.

12. The medical support device according to claim 10,
wherein the insertion route is indicated by a line.

13. The medical support device according to claim 7,
wherein, the medical support information is insertion support information of a treatment tool (18) that is inserted from outside the body toward a target position in the organ observed through the medical probe, and is the insertion support information including at least one of an insertion position or an insertion route,
the medical probe is provided in the insertion portion and has a guide groove (29) for guiding insertion of the treatment tool into the target position by engaging with the treatment tool, and
the processor (41) is configured to specify a position at which the insertion support information is superimposed on the captured image based on a relative positional relationship between the second marker and the guide groove (29).

14. The medical support device according to claim 8,
wherein the processor (41) is configured to specify a position at which the insertion support information is superimposed on the captured image based on the target position specified in an internal image of the organ acquired by the medical probe, and a correlation between a coordinate system of the internal image and a coordinate system of the captured image which are derived based on the position and posture information.

15. The medical support device according to claim 7,
wherein, the medical support information is insertion support information of a treatment tool (18) that is inserted from outside the body toward a target position in the organ observed through the medical probe, and is the insertion support information including at least one of an insertion position or an insertion route,
in a case where the insertion support information is set in a three-dimensional image of the organ acquired in advance before surgery by simulation before the surgery, and
the processor (41) is configured to superimpose the insertion support information on the first captured image (23) using a correlation between a coordinate system of an internal image of the organ acquired by the medical probe and a coordinate system of the three-dimensional image, and a correlation between a coordinate system of the internal image and a coordinate system in the second captured image which are derived based on the second position and posture information.

16. The medical support device according to claim 8,
wherein the extracorporeal camera (15) is provided on a proximal end side of the treatment tool (18) and is configured to output the captured image as a video image, and
the processor (41) is configured to display a line indicating the insertion route in the video image as the insertion support information.

17. The medical support device according to claim 16,
wherein the treatment tool (18) is a puncture needle, and an imaging optical axis of the extracorporeal camera is disposed along an axial direction of the puncture needle.

18. The medical support device according to claim 8,
wherein the captured images are at least two captured images captured from different viewpoints, and
the processor (41) is configured to derive the insertion position on the body surface of the subject by obtaining an intersection of the insertion routes shown in the two captured images.

19. An operation method of a medical support device including a processor, the operation method comprising:
via the process,
acquiring a captured image (23) that is captured by an extracorporeal camera (15) provided outside a body of a subject and in which a medical device (13) whose insertion portion (13A) is inserted into the body of the subject and a marker (M1) which is provided at a portion of the medical device excluding the insertion portion and is image-recognizable are included in an imaging range;
deriving position and posture information including at least one of a position or a posture of the medical device in the captured image (23) based on the marker (M1); and
executing a control of displaying, on a display unit (16), a composite image in which medical support information is superimposed at a position specified in the captured image (23) based on the position and posture information.

20. A computer-readable storage medium storing a operation program of a medical support device that causes a computer to function as a medical support device, the operation program causing the computer to:
acquire a captured image (23) that is captured by an extracorporeal camera (15) provided outside a body of a subject and in which a medical device (13) whose insertion portion (13A) is inserted into the body of the subject and a marker (M1) which is provided at a portion of the medical device excluding the insertion portion and is image-recognizable are included in an imaging range;
derive position and posture information including at least one of a position or a posture of the medical device in the captured image (23) based on the marker (M1); and
execute a control of displaying, on a display unit (16), a composite image in which medical support information is superimposed at a position specified in the captured image based on the position and posture information.
